# EUROPEAN PATENT SPECIFICATION

(11) **EP 4 658 070 B1**
(45) Date of publication and mention of the grant of the patent: **28.10.2026**
(21) Application number: 24706902.4
(22) Date of filing: 18.01.2024
(51) Int. Cl.: A01N 1/143, A61B 17/122

(54) **CANNULA FOR CONNECTING TO AND INTERFACING WITH AN ORGAN**
KANÜLE ZUR VERBINDUNG MIT EINEM ORGAN UND ZUR VERBINDUNG MIT EINEM ORGAN
CANULE POUR CONNEXION À UN ORGANE ET MISE EN INTERFACE AVEC CELUI-CI

(30) Priority: 31.01.2023 US 202363482383 P; 05.07.2023 US 202363511995 P
(43) Date of publication of application: 10.12.2025
(60) Divisional application: 26154771.5 / 4 755 183; 26154772.3 / 4 755 184
(73) Proprietor: DEKA Products Limited Partnership, Manchester, NH 03101 (US)
(72) Inventor: MOREAU, Timothy D., Manchester, NH 03104 (US); STADNICKI, Connor E., Epsom, NH 03234 (US); LANIGAN, Richard J., Concord, NH 03301 (US)
(74) Representative: EIP
(86) International application number: PCT/US2024/011999
(87) International publication number: WO 2024/163184

(56) References cited:
- CN-A- 112 806 351
- CN-U- 214 903 403
- US-A- 5 928 253
- US-A- 6 042 563
- US-A1- 2011 212 431
- US-B1- 6 673 594
- US-B1- 6 953 655

## Description

### BACKGROUND

The present disclosure relates to maintenance, assessment, maturation, and rehabilitation of a tissue for a transplant recipient. More specifically, the present disclosure relates to an interface for connecting with the vessels of a tissue in a nondestructive way to allow connection of that tissue to a tissue life support system.

In 2020, there were about 39,000 tissue transplants of all types of tissues performed in the United States. Every nine minutes, a new patient is added to the transplant waiting list. There are a number of different types of tissues that are utilized from organ donors. Tissues, including organs, that are considered for transplantation include, but are not limited to, kidney, pancreas, liver, heart, lung, stomach, intestine, composite allografts, thymus, uterus, skin, bone, tendon, middle ear, cartilage, heart valves, trachea, nerves, veins, hands, feet, arms, adrenal tissue, fetal thymus, cornea, and composite transplantation tissue. Regarding specific organs, in the United States in 2020, there were 12,141 people on the liver transplant waiting list, while only 8,906 transplants occurred. Pancreas, heart, lung, and intestine candidates awaiting transplants numbered 14,489. Further, while there are 91,834 kidney candidates on the transplant list, only around 20,000 kidney transplants performed.

With respect to the kidney, in 2020, over 700,000 patients per year in the United States and an estimated 2 million patients worldwide were affected by end stage renal disease (ESRD). Further, morbidity among ESRD patients increased during the early months of the COVID-19 pandemic resulting in an estimated 6,953-10,316 deaths. The primary treatments for ESRD are dialysis and kidney transplant. In the United States, an overwhelming percentage of people with ESRD are on dialysis, with a small fraction living with transplants. In general, patients on dialysis have a lower life expectancy and lower quality of life than patients receiving a kidney transplant. Most transplanted kidneys come from deceased donors, and yet a large number of these available kidneys are discarded. Deceased donor kidneys have several challenges: higher rates of delayed graft function (DGF) post-transplant, longer warm ischemic time during kidney recovery, higher degree of cold induced injury, and lower long term graft survival rates.

In harvesting tissue for transplantation, supporting tissues that feed necessary life support to the tissue as well as providing necessary connective tissue during transplantation are cut in a manner that leaves intact as much of the supportive tissue as possible. During storage and transplantation, support means are often connected, removed and reconnected to the supportive tissue to facilitate feeding life supportive nutrients to the tissue to be transplanted. During such connection, removal and reconnection, often incremental portions of the supportive tissues are cut or altered thereby reducing the supportive tissue available for latter attachment during transplantation.

Improved options for preservation of the tissues awaiting transplantation include normothermic/subnormothermic perfusion, which is likely to extend preservation time, enable real time tissue diagnostics, and significantly reduce cold-induced injury. Preservation techniques such as *ex vivo* normothermic machine perfusion (NMP) can be used to assess the quality of the tissue before transplantation before committing the recipient to surgery. Normothermic or subnormothermic perfusion results in a metabolically active tissue, which can enable assessment via direct measures of kidney function and through enabling laboratory analysis of tissue and perfusate samples.

There is therefore a need for a system designed to reduce the number of discarded tissues. There is a further need for a system that facilitates interfacing with transplantation tissue in a manner that maintains a pre-selected oxygen level in circulating perfusate and/or that can constantly monitor the tissue and dynamically adjust the desired oxygen level, possibly during transport from the donor to the transplant recipient. Still further, a system is needed that can provide a releasable and damage free interface with a tissue that allows the necessary nutrients to flow to the tissue to maintain its vitality before transplantation, while preserving supportive tissues in an undamaged manner to allow better success during future transplantation while still allowing interface with the transplantation tissue to sense a sufficient range of characteristics to help medical personnel decide whether or not the tissue is viable, for example, but not limited to glucose and pH. A successful tissue maintenance and assessment system can provide medical personnel with quantitative measures of tissue health, enable reconditioning of the tissue to optimize its performance prior to implant, and enable *ex vivo* treatment of the tissue, for example, but not limited to, pharmacologic and gene therapy. What is further needed is a system that achieves low hemolysis and maintains desired characteristics of the tissue. What is needed is a normothermic/subnormothermic tissue perfusion device with onboard sensors to simulate body circulation and monitor the tissue, respectively. What is needed is a device that enables, releasable interface, output flow, sampling, and recirculation.

CN 112806351 B relates to an organ perfusion device, a transport system having the perfusion device, and a perfusate. The perfusion device for the donor heart includes a sterile field formed by a plurality of flexible membranes; a housing formed by a plurality of rigid structures in which the plurality of flexible membranes are located; and a first connector for securing and combining the donor heart. the aorta of the donor heart; a second connector located at the bottom of the flexible membrane for diverting perfused fluid away from the donor heart; a first conduit structure located between the housing and the flexible membrane and connected to the first connector is connected, and a flow sensor, an oxygen content sensor and a temperature sensor are arranged in the first conduit structure; a display screen, a storage device and a controller are arranged on the casing. The perfusate temperature adjustment device, oxygenation device, flow sensor and pressure sensor connected to the first conduit. The perfusion device can monitor the perfusate and protect the donor heart. CN 214903403 U describes an isolated perfusion heart fixing and clamping device. Including a perfusion tube connector, a positioning and clamping assembly, and a cannula. The cannula is fixedly connected to the perfusion tube connector, and the positioning and clamping assembly is movable is provided at the upper end of the cannula, the lower end of the cannula is provided with a slot, the positioning and clamping assembly includes a positioning sleeve and a clamping piece arranged on the positioning sleeve, the clamping piece and the positioning sleeve cooperate with each other. The aorta is fixed in the slot.

### SUMMARY

The invention relates to a cannula in accordance with claim 1.

Preferred embodiments are set out in the dependent claims. The present teachings include an interface system and method for tissue perfusion in order to maintain and possibly rehabilitate the tissue. Among other features, the present teachings provide an interface for interconnection of transplantation tissue with a support system that can include a tissue enclosure having a fluid reservoir. Pumps, valves, and a controller can move perfusate through the tissue via the interface. The support system can include features to assist in monitoring the health of the tissue, and a removable tray to facilitate moving the tissue from a point of origin to the tissue enclosure. The system of the present teachings is fully configured to perfuse and provide nutrition for transplant tissue such as human tissue. Other features of the system of the present teachings include, but are not limited to, a urine flow rate sensor, a nutrition pump, disposable and durable parts, and noncontact (with the perfusate) sensors.

The system for perfusing and organ of the present teachings can include at least one controller or processor that can enable valves and pumps to perfuse fluids through tissue, for example, but not limited to, a human tissue. The at least one controller/processor can be, for example, but not limited to, a general purpose processor managing several tasks, a custom processor configured to manage a specific task, a proportional controller, and integral controller, a derivative controller, a programmable logic controller, a distributed control system, a programmable automated controller, a microcontroller, a microprocessor, an embedded processor, or supervisory control and data acquisition software. The controller/processor can receive data from sensors located throughout the system and other forms of data input, and can adjust, among other things, the pumps and valves according to the data. For example, the controller/processor can receive user input, recipe input, and/or default settings that can be used, along with values of the sensor data, to adjust the flow parameters of the perfusate. In addition to controlling perfusate flow, the controller/processor can issue instructions to pumps and valves that regulate the provision of infusion/nutrition to the tissue. In some configurations, infusion/nutrition can be pumped into the fluid in a fluid reservoir that is in fluidic communication with the tissue. The contents of possible infusion/nutrition options can be pumped into the fluid reservoir when sensors indicate a need for
perfusate modification. The system of the present teachings can include a pumping cassette that can deliver, under direction from the controller/processor, the nutrition/infusion at a possibly variable rate specific to a particular tissue, and specific to the current status of the tissue. The controller can perform processing associated with the specific sensor suite. In some configurations, the controller can automatically determine which sensors are available in a particular system and execute processes associated with the identified sensors.

The system of the present teachings can include a pump subsystem that can enable tissue perfusion and perfusate recirculation. The pump subsystem can pump perfusate, for example blood and other additives, through the tissue. The blood can include whole blood or packed red blood cells, for example. In some configurations, the pump subsystem can enable perfusate flow at a rate of up to 600ml/min at a pressure of 20-120mmHg. Flow can optionally be pulsatile, and the rate can be adjustable. As an example, a low flow rate can be required for cold or damaged tissue. As tissue function improves, the flow rate can be adjusted to accommodate the changed conditions. Pulsatile flow or a flow rate controlled by physiological parameters can both be accommodated by the pumps of the present teachings. One goal of pump choice is to reduce hemolysis. Direct acting pneumatic pumps can enable minimal hemolysis and flow metering for wetted materials as discussed herein. It can be possible to modify the pumping cycle of direct acting pneumatic pumps to match physiological pulsatile pressure duty cycles.

The role of the perfusion loop is to provide basic biological functions that would otherwise take place in the body. These include oxygenation, nutrient supply, thermal control, and removal of carbon dioxide. Oxygenation and removal of carbon dioxide are conducted through the use of a membrane oxygenator. A heat exchanger is used to maintain desired perfusate temperature. The perfusion fluid leaves the tissue, is passed through an oxygenator, is passed through a heat exchanger, and is then pumped back into the tissue. Nutrients are suppled in the perfusion solution and can be added manually or through the use of automated infusion pumps. Output generated by the tissue flows out of the tissue and is available for sampling through sterile sample ports. Output can be directed back into the perfusion loop or discarded. Output flow rates and volumes are measured and stored by the system. In the event that recirculating output proves to be a challenge, the system can be modified such that the output is collected or potentially passed through a dialysis loop.

The perfusion loop acts like a maintenance loop for the system allowing for filling or draining of the fluid reservoir and recirculation of the fluid from the tissue reservoir, essentially stirring the tissue reservoir. This loop can include the infusion pumps so that infusions can be delivered, diluted, and mixed into the perfusate instead of being passed directly into the tissue. Some or all of the infusion pumps can be made part of the perfusion loop. In some configurations, the system includes a bypass valve that can be opened during priming when bubbles are detected. To introduce new blood or drain the system, the system includes at least one valve associated with the infusion path. In some configurations, a pinch valve can be associated with incoming perfusate, while another pinch valve can be associated with a drain path. In an aspect, pneumatic valves can be used. Other types of valves are contemplated by the present teachings. The perfusate pump can also drain the tissue enclosure.

The system includes means for monitoring, for example, the tissue, the perfusate, and the tissue's output. The data collected during monitoring can be used to adjust, for example, the environment of the tissue and the characteristics of the perfusate. Any types and numbers of sensors can be used for monitoring, and the controller can be programmed to automatically or manually respond to an instantaneous situation. In some configurations, the type of nutrition provided, the dissolved oxygen in the perfusate, the blood oxygen saturation level, the perfusion pumping rate, the glucose, the temperature, the pH, and/or the CO₂ are monitored through a group of sensors strategically positioned in the perfusion loop. The system includes sensors to enable adequate perfusion and collect data for tissue assessment, sterile sample ports for removing output and perfusate fluids using a sterile syringe, and sensors that are out of the fluid path as well as sensors that are in the fluid path.

The tissue enclosure provides a barrier to contamination for the tissue as it is being maintained. In an aspect, the tissue enclosure includes three main parts - a fluid reservoir, a tissue platform, and a hood, all operably coupled to form an isolation environment for the tissue. The geometry of the tissue enclosure includes connectors to receive the tissue platform, connectors and seals to receive the hood, a well to house the fluid, an air space above the fluid in which the tissue platform is placed, and a fluid ramp receiving at least some of the tissue output and channeling the output towards the fluid reservoir. A temperature control mechanism is positioned in the vicinity of the tissue enclosure. In an exemplary configuration, the temperature control mechanism is positioned beneath the tissue enclosure, and is fluidically coupled with the tissue enclosure.

In an aspect, the fluid reservoir receives, for example, but not limited to, output products from the tissue, venous output from the tissue, and possibly nutrition and medications. The types and amounts of components in the fluid are not limited to such additives as are listed herein, but instead include components that are appropriate for the type of tissue being maintained. The fluid reservoir sits below the tissue platform, and thus, the tissue resides in the air space above the fluid reservoir. Perfusate is pumped from the fluid reservoir and its characteristics and temperature are adjusted and monitored before it is pumped into the tissue.

The tissue platform can optionally be partitioned into areas of the platform floor. One area can be configured to receive the tissue. The tissue itself can be positioned on the platform floor on which it can be cannulated or otherwise operably coupled with perfusion tubes. In some configurations, the tissue is secured to the platform by, for example, a strap, tie-down, belt, or cord anchored in depressions on the rim of the tissue platform. Part of the floor can be configured to manage the tubing or possibly cabling. Tube management can include, but is not limited to including, weld mount clamps, rail clamps, magnetic clamps, snap-in clamps, multiline clamps, connectable clamps, expansion clamps, adhesive-back clamps, lock-close strut-mount clamps, standoff clamps, low profile clamps, and loop clamps. In an exemplary configuration, tubes and cables are routed through merlons positioned on the floor of the tissue platform, spaced according to, for example, the expected sizes of tubes and cables. Other tube and cable mount points enable the tubes and cables to be raised above the floor of the platform. In an exemplary configuration, one or more standoff features is configured with tube holders such as bent finger-like projections. In an exemplary configuration, the tubing and cables are routed along the sides of the tissue platform between crenellated edges to enable tube/cable routing between the tissue platform and other parts of the tissue maintenance system. The crenellations can be spaced according to a desired or expected tube/cable size. Multiple tubes/cables can be accommodated by the spaces between the crenellations, if desired. The tubing or cabling is routed to exit the tissue platform by, for example, but not limited to, ductwork, routing tubing, routing panels, or channeling. The tubing is coupled with connectors that enable connection to further tubing in the tissue enclosure. The on-board coupling between the tissue and the perfusion tubes or other required system connections makes it possible to ready the tissue for management by the system remotely from the tissue enclosure and convenient to the location of the tissue. This can reduce the amount of manual manipulation the tissue has to endure.

Other platform configurations are contemplated by the present teachings. The platform, detachable from the fluid tank, can be the only part of the system that could be specific for a tissue type, although platforms are contemplated to be used for multiple tissue types. The platform described herein, used for a kidney, illustrates the features of a particular platform. The present disclosure is not limited to accommodating a kidney platform, nor to the geometry of the kidney platform.

In an aspect, the tissue enclosure includes a hood that includes a durable/disposable barrier and houses at least one sensor. The barrier and gasket fitted to the tissue enclosure protect the tissue on the tissue platform from external environmental conditions. The barrier enables manual and automatic observation of the tissue on the tissue platform, and is securely attached at the rim of the tissue enclosure to fully shelter the tissue while still providing viewing options. Observation can include providing images of the tissue, which can aid a user in assessment of the tissue. For example, the tissue can be measured, the color of the tissue can be observed to detect, for example, free hemoglobin or bacterial infection, and the size/shape over time of the tissue can be determined to detect, for example, if the tissue is undergoing edema. If the tissue performs a particular physiological function, the user and/or the controller can observe the function of the tissue over time.

The system of the present teachings pumps perfusate in a closed loop through the tissue. In an aspect, the system includes one or more fluid pumps to accomplish the perfusion. Types of perfusion pumps include, but are not limited to including, axial flow pumps, peristaltic pumps, diaphragm pumps, pumping cassettes, roller pumps, centrifugal pumps, pulsatile pumps, and non-occlusive roller pumps. A pump that can enable the perfusion of the system of the present teachings can deliver physiologic blood flows against high resistance without damaging blood, provides flows that are exact and easily monitored, creates no turbulence or stagnation, and can be manually operable in the event of a power failure. In some configurations, extracorporeal membrane oxygenation (ECMO)-type devices are used to perfuse and oxygenate the blood in the system. In some configurations, the oxygenator device uses silicone membrane contactors. The perfusate is pumped through several possible modification stations and past several sensors before entering the tissue. In an exemplary configuration, a pumping cassette, at the direction of the controller, can move perfusate from the fluid reservoir into an oxygenator.

The tissue receives perfusate into a cannulated orifice of the tissue, and produces output through another cannulated orifice of the tissue. For example, if the tissue is a kidney, at least one of the outputs is urine. At least one output from the tissue is routed from the tissue for monitoring of the output before the output is routed back into the fluid reservoir or is routed to a waste area. To enable monitoring of the output, the system includes an output flow device that includes a collector container and sensors and a means for managing the collection device accumulation. The container can take any shape and can, for example, include graduated fill marks. Convenience of mounting the container with respect to the platform and output measurement criteria can be considered when choosing a container shape and size. The container includes at least one sensor that indicates, to a controller, the level of the output in the container. In some configurations, the container is coupled with a plurality of sensors, at least one at a desired output high level, at least another at a desired output low level. When the output reaches the high level sensor, the controller directs the valve to open to release the output. When the output reaches the low level sensor, the controller directs the valve to close, thereby retaining the fluid in the container again. In an exemplary configuration, the level sensor includes an ultrasonic sensor. In an aspect, the level sensor includes at least one visual sensor that determines the level of the fluid by locating floats within the fluid. The present teachings contemplate multiple high and low levels to enable various types of measurements. The valve includes, but is not limited to including, being selected from ball, butterfly, check, gate, knife gate, globe, needle, pinch, and plug valves. In an exemplary configuration, the valve is a pinch valve. In an aspect, the valve is a pneumatic valve. In some configurations, the output flow device is configured so that visual inspection of the output is possible. For example, the output passes through a transparent or partially transparent container. The container can be fully opaque except for a window, or can be substantially transparent, or some layout in between.

The system of the present teachings provides nutrition and medications, for example, to the tissue when required. The controller controls a device that accesses various infusion materials, depending on the needs of the tissue. Nutrients and medications can include, but are not limited to including, water, lipids, amino acids, glucose, vitamins, hormones, antibiotics, chemotherapy drugs, vasodilators, vasoconstrictors, diuretics, antidiuretics, anticoagulants, and insulin. In an aspect, a kidney's characteristics are controlled by regulating infused substances, monitoring the results of the infusions on the characteristics of the kidney, and then adjusting the infusion rate based on the results. Nutrients are provided by infusion pumps, devices that deliver nutrients and medications in controlled amounts. The pump is configured, either automatically or manually, to provide specific nutrients at a specific flow rate. Automatic infusion configuration occurs when the system of the present teachings determines which kind of tissue is being processed and sets the nutrition and medication regime automatically. Manual infusion configuration occurs when the system accesses, or a user provides, set-up parameters such as the components of the nutrients and/or medications, and the delivery rate and times of delivery. In any case, the controller determines when there is a potential or actual pump failure, or when there is a potential or actual drug interaction problem, among other types of alerts. In an exemplary configuration, nutrition and medication are pumped into the fluid reservoir by a fluid pump, the possibilities of which are described herein. In an exemplary configuration, a pumping cassette pumps the nutrition and medication to the tissue, either directly into the arterial line or into the reservoir. In an aspect, the pumping cassette is sized to accommodate the requirements of supplying nutrition/medication. For example, the pumping cassette includes a single chamber that pumps a pre-selected amount of fluid at a consistent rate, such as 10ml/hour. In an aspect, sensors measure the consumption rate of the nutrients/medications, and those sensor data are used to control the infusion of those components. For example, a glucose sensor measures how much glucose is in the perfusate exiting the tissue, and that measurement is used to adjust the amount of infused glucose based on the metabolic rate of the tissue. The nutrition pump of the present teachings is configured to pump from an intravenous bag at rates of 1-20 mL/hour, removing the need for an IV pump. In an aspect, the nutrition pump is a sterile disposable device that is integratable with the pneumatics of the present teachings. In an aspect, the nutrition pump runs closed loop glucose control.

In some configurations, a low bolus, high accuracy infusion pump is used to enable clinical infusions such as prescription vasodilators or insulin. In some configurations, multiple infusion pumps are used to enable multiple different substances to be infused, possibly simultaneously. In some configurations, the pump reservoir is 3mL, the pump accommodates an infusion rate of 0.5-300.0µL/hr, an infusion volume of 0.5-250.0µL, and infuses into a recirculation loop that feeds into the tissue enclosure.

The method of the present teachings can include, but is not limited to including, mounting the tissue on the tissue platform, preparing it for connection to the fluid tank. The method can include coupling the tissue's orifices with pre-selected locations on the platform through tubing, connectors, and the like. For example, if the platform is configured for a kidney, the platform can include a connector and tubing to transport perfusate into the kidney and another connector and tubing to transport urine out of the kidney. The method can include directing the venous output into the fluid reservoir. The vein can be cannulated and directed by tubing to the fluid reservoir, or can simply exit the kidney and pass through a cavity in the platform to the tissue reservoir. In an exemplary configuration, the artery and the ureter can be cannulated, and the cannulation tubing can be fed through protrusions on the platform that can prevent movement of the tubing on its way to the platform orifices and connectors.

In one aspect, the means for coupling to the tissue orifices includes a cannula configured and arranged at one end to interface with the tissue and at an opposing end to interface with the tubing of the perfusion loop. The cannula includes at least one cannula post with a lumen extending there through such that the cannula post is inserted into a vessel of the tissue. For example, if configured for a kidney, the cannula post can be inserted into the artery, vein or urethra of the kidney and connected at the opposing end to the tubing of the perfusion loop. The cannula may include over molded regions to both soften the contact regions of the cannula and provide positive sealing as between the vessel of the tissue and the cannula. The cannula may also include retractable locking collars to clamp, retain and seal the vessel of the tissue onto the cannula post.

In another aspect, the means for coupling to the tissue orifices includes a cannula configured and arranged at one end to interface with the tissue and at an opposing end to interface with the tubing of the perfusion loop. The cannula includes at least one annular pad with a lumen extending there through such that a terminal end of a vessel of the tissue is received adjacent the annular pad. For example, if configured for a kidney, the terminal end of the artery, vein or urethra of the kidney is received in contact with the annular pad and connected at the opposing end to the tubing of the perfusion loop. The cannula may include over molded regions to both soften the contact regions of the cannula and provide positive sealing as between the vessel of the tissue and the cannula. The cannula may also include retractable locking collars to clamp, retain and seal the vessel of the tissue onto the annular pad.

In some aspects, the means for coupling to the tissue includes a displacement means to move the locking collars in a manner that clamps the tissue orifice in contact with the annular pad and/or cannula post.

In some aspects, the annular pad and/or cannula post, and the locking collars may be configured to receive more than one vessel of the tissue.

In another aspect, the means for coupling to the tissue orifices includes a cannula configured and arranged at one end to interface with the tissue and at an opposing end to interface with the tubing of the perfusion loop. The cannula includes at least one annular pad with a lumen extending there through. One or more optional cannula posts, each having a flared end may be positioned in contact with the annular pad such that a lumen there through aligns with an opening in the annular pad. One or more vessels of the tissue is received onto the one or more cannula posts. The cannula may also include retractable locking collars to clamp, retain and seal the vessel of the tissue and the optional cannula post(s) onto the annular pad.

The method can include coupling the platform connectors to the perfusion system and clicking the platform into place atop fluid in the fluid reservoir. The fluid tank can include space for fluid below the platform. Into this fluid can flow the output from the tissue mounted on the platform. For example, if the tissue is a kidney, the output is venous perfusate and urine.

The system of the present teachings includes a combination of disposable and durable materials. For example, the oxygenation means is disposable, along with the heat exchanger, while the thermal energy source is durable. The at least one perfusion pump is disposable, while the at least one pump interface coupling the at least one disposable pump with the pneumatics is durable. The at least one infusion pump is disposable. The pneumatics can optionally include at least one durable valve, at least one durable chamber, at least one durable pressure source, and at least one durable vacuum source. Durable components can include at least one sensor providing sensor data monitoring the tissue, and at least one controller receiving and processing the sensor data. Disposable components can include spot sensors, tubing, cannulas, cassette pumps, tissue containers, and the oxygenator.

### BRIEF DESCRIPTION OF THE DRAWINGS

The foregoing features of the disclosure will be more readily understood by reference to the following description, taken with reference to the accompanying drawings, in which:
FIGs. 1A-1F are schematic block diagrams of the system of the present teachings for maintaining and assessing tissue;
FIGs. 2A-2V are schematic perspective, elevations, and top/bottom/side views of a configuration of one cannula of the present teachings;
FIGs. 3A-3E are schematic perspective, elevations, and top/bottom/side views of a configuration of an alternate cannula of the present teachings;
FIGs. 4A-4E are schematic perspective, elevations, and top/bottom/side views of a configuration of another alternate cannula of the present teachings;
FIGs. 5A-5D are schematic perspective, elevations, and top/bottom/side views of a configuration of yet a further alternate cannula of the present teachings;
FIGs. 6A-6B are schematic perspective, elevations, and top/bottom/side views of a configuration of another alternate cannula of the present teachings; and
FIGs. 7A-7C are schematic perspective, elevations, and top/bottom/side views of a configuration of a further alternate cannula of the present teachings.

### DETAILED DESCRIPTION

The system of the present teachings for maintaining, assessing, maturing, and rehabilitating tissue is described in detail herein. In this regard, the present disclosure relates to maintenance, assessment, maturation, and rehabilitation of a tissue for a transplant recipient. More specifically, the present disclosure relates to an interface for connecting with the vessels of a tissue, in a non-destructive way, to allow connection of that tissue to a tissue life support system. The system and method of the present teachings is configured to provide a releasable, non-destructive interface for the interface with a tissue in order to allow for real-time assessment of the tissue, using that assessment to continuously maintain the health of the tissue. The system of the present teachings includes, but is not limited to including, a disposable set of components and a durable set of components. The disposable components include, but are not limited to including, a tissue container assembly holding the tissue and a reservoir of perfusate, a perfusion pump assembly pumping perfusate through the tissue, tubing and tissue interface connecting the tissue container assembly with the perfusion pump assembly, a tissue gas adjustment device maintaining a myriad of characteristics of the perfusate, and sensors providing data about the tissue necessary to maintain the tissue. Disposable components can also include at least one infusion pump assembly providing nutrition and medication to the tissue, and an output monitoring, measuring, and sampling assembly receiving output from the tissue, assessing the output in real-time and possibly off-line, and returning the output to the tissue container perfusate reservoir. The durable components include, but are not limited to including, a tank monitor assembly enabling protection of the tissue from environmental contamination as well as visual inspection and sensory recording of the tissue, a thermal adjustment assembly maintaining the temperature of the perfusate, a pneumatics assembly driving the perfusion pump assembly to circulate the perfusate, power, data, and control electronics energizing the components of the system and sequencing events in the system based at least on sensor data.

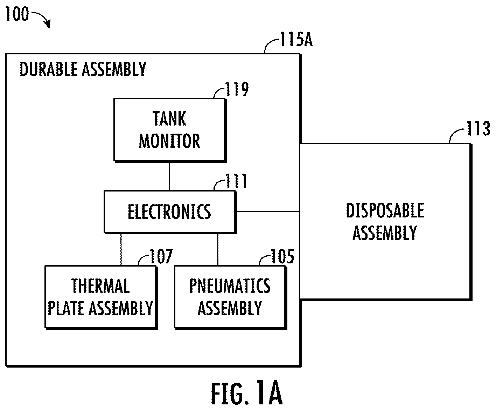

Referring now to FIGs. 1A-1F, various configurations of a system for maintaining, assessing, maturing, and rehabilitating tissue are illustrated in block diagram form. All exemplary configurations include durable and disposable assemblies. The present teachings contemplate further configurations than those depicted herein. The drawings in FIGs. 1A-1E are for illustration purposes only. In an aspect, system 100 (FIG. 1A) of the present teachings includes durable assembly 115A (FIG. 1A) and disposable assembly 113 (FIGs. 1A-1D). Disposable assembly 113 (FIGs. 1A-1D) includes a container or tank that houses the tissue that is to be maintained and assessed. The container, while being operably coupled with durable assembly 115A (FIG. 1A), protects the enclosed tissue and fluids nourishing and medicating the tissue from environmental contamination. Durable assembly 115A (FIG. 1A) includes tank monitor 119 (FIGs. 1A-1D), electronics 111 (FIGs. 1A-1D), thermal adjustment assembly 107 (FIGs. 1A-1D), and pneumatics assembly 105 (FIGs. 1A-1D). Tank monitor 119 (FIGs. 1A-1D) includes at least one sensor that can capture and retain data about the tissue resting in disposable assembly 113. Tank monitor 119 (FIGs. 1A-1D) includes, for example, a transparent barrier that can at once enable inspection of the tissue while at the same time protecting the tissue from environmental contamination. The barrier can be completely transparent to all frequencies, or completely transparent to some frequencies and opaque to others. Parts of the barrier can be opaque, while others can be transparent. Inspection can be performed manually, manually enabled by sensors, partially automatic, or completely automatic through controller-enabled sensors. Sensors can include, but are not limited to including, cameras and x-rays, and remote probes that monitor temperature, humidity, light, pressure, air quality, and differential air pressure, for example. Electronics 111 (FIGs. 1A-1D) include a controller that manages various activities with respect to the sensors, for example, collecting, displaying, analyzing, and storing data from the sensors. Thermal adjustment assembly 107 (FIGs. 1A-1D) maintains a desired temperature within disposable assembly 113 (FIGs. 1A-1D), providing thermal regulation for a perfusate that is used to nourish and medicate the tissue without coming into contact with the tissue. Moving perfusate, nutrition, and medication to and through the tissue in enabled by pneumatics assembly 105 (FIGs. 1A-1D) which drives at least one disposable pump. Other methods to drive the pump(s) are contemplated by the present teachings. Pumps in the system of the present teachings have delivery requirements. These requirements establish characteristics that are desired by any device that drives the pumps. In an aspect, a pneumatic valve assembly can deliver the quantities required without damaging the traversing fluid.

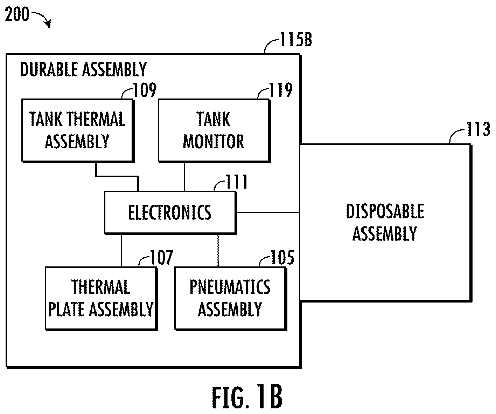

Referring now to FIG. 1B, exemplary system 200 includes durable assembly 115B which includes tank thermal adjustment assembly 109. In such a configuration, tank thermal adjustment assembly 109 insulates the tank and provides thermal control of the tank. The purpose of the assembly is to substantially prevent condensation from adhering to the tank. Condensation on the tank can affect the monitoring of the tissue, and can be indicative of a condensation/vaporization cycle that causes the tissue to lose moisture or causes the tissue surface to dry out, which can impact the viability of the tissue. Insulating the tank can reduce condensation, and thermal control can reduce condensation as well.

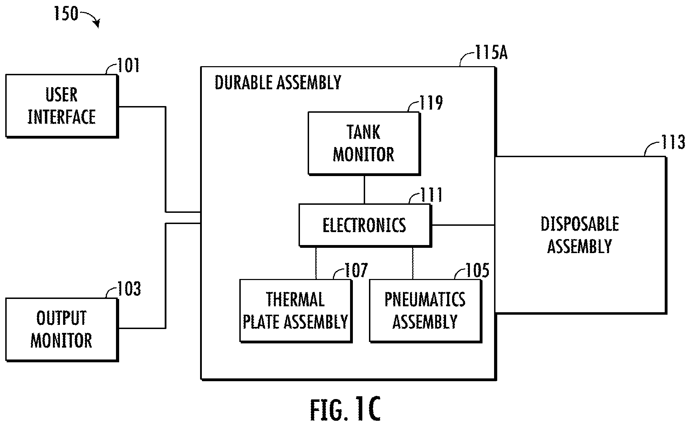

Referring now to FIG. 1C, exemplary system 150 includes user interface 101 and output monitor 103. In such a configuration, the sensor data gathered by sensors in durable assembly 115C and disposable assembly 113 can be made available to a user through user interface 101. Possible user interface options include wired and wireless devices, devices with and without visual interface, audio interface, and tactile interface, and/or a combination of interfaces. For example, a computer monitor can display a read-out of sensor data gathered with regard to the tissue, and can be coupled with a keyboard in which the user can request types of data and/or control the sequencing of events occurring with respect to the tissue. The user interface can be used to entirely or partially override any automatic behavior that the system takes to maintain the tissue, or augment such behavior. Changes in the tissue over time can be graphically depicted, either by tables of tissue characteristics as they change over time, graphical depictions of such data, photographs and/or videos of the tissue at various check points or continuously, an audio report of the tissue, and/or a tactile read-out of the situation. Data can be analyzed and logged, and the user and/or system can retrieve the analyzed data. Output monitor 103 enables collecting output from the tissue, measuring it, inspecting it, and routing it based on automatic and/or manual selections. Output monitor 103 includes a vial into which tissue output is routed by, for example, tubing cannulated to an orifice of the tissue, for example, a ureter if the tissue is a kidney. Other types of routing of the output are contemplated by the present teachings. In any case, the tissue enclosure is properly sealed from environmental contaminants even as the output travels from inside the enclosure to a collection point outside the enclosure. Connectors that provide for environmental isolation and a closed circulation path maintain the desired protection from contaminants. The output travels to a collection point in which the output can be measured, assessed, and released. The collection point can allow for assessment including manual visual inspection, through fully automatic multi-sensor assessment, and all types of inspection and assessment in between. For example, the user can visually inspect urine if the collection point is configured as a transparent or semi-transparent container, if the tissue is a kidney, and manually adjust parameters that can bring the urine back to a healthy appearance. Likewise, sensors can automatically deliver data about the urine to the controller, and the controller can adjust parameters automatically that affect the health of the kidney. In an aspect, the collection point includes at least one incoming fluid path that admits output from the tissue into the collection point. In an aspect, the collection point includes a means for detecting the volume of output. When a pre-selected amount of output is collected, the amount is measured and released through at least one outgoing fluid path. In an aspect, the means for detecting volume includes at least one level sensor coupled with at least one valve controlled by the controller. In an aspect, the controller receives a signal when a level sensor detects that the fluid has reached a pre-selected level in the collection device. In an aspect, the controller activates sensor(s) to examine the collection of output. When the examination is complete, the controller opens at least one valve and releases the output. The controller discontinues the release by closing the valve when a level sensor detects that the fluid has reached a pre-selected level in the collection device. Other means for measuring the level of output are contemplated by the present teachings. The output can be released to at least one reservoir. In an aspect, the output can be released to a reservoir selected by the controller. In an aspect, a single outgoing output path can be routed through a multi-path connector. In an aspect, one path can route the fluid into the tissue container to join with the reservoir of perfusate. In an aspect, one path can route the fluid into a waste reservoir that can be removed and deposited in an appropriate receptacle. In an aspect, one path can route the fluid into a sample reservoir that can be removed and assessed offline. Other output paths are contemplated by the present teachings.

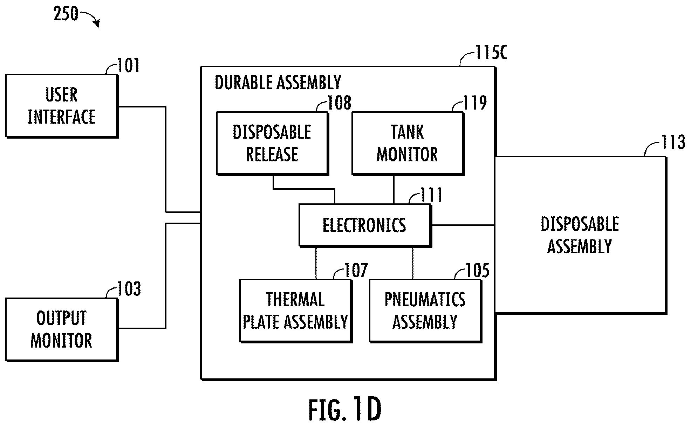

Referring now to FIG. 1D, durable assembly 115C of exemplary system 250 can include disposable release 108. In an aspect, disposable assembly 113 can be completely decoupled from durable assembly 115C by (1) aligning complementary fittings, such as between durable pneumatic assembly 105 and the disposable pumps controlled by pneumatic assembly 105, and (2) engaging a mechanism that enables secure coupling between the disposable and durable assemblies. In an aspect, the mechanism includes at least one tapered locking shaft coupled with at least one disposable locking carriage. The locking carriage locks disposable assembly 113 in place by sliding across the locking shaft until a spring plunger is cleared. To decouple disposable assembly 113 from durable assembly 115C, the spring plunger is released. Other means of coupling durable and disposable assemblies are contemplated by the present teachings.

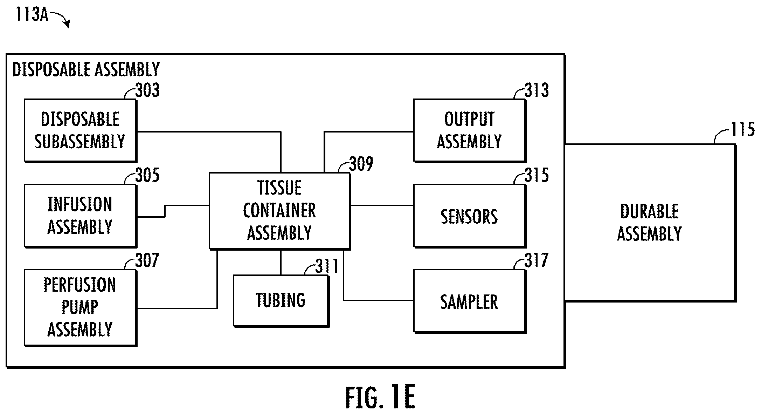

Referring now to FIG. 1E, disposable assembly 113A includes components that have direct contact with the tissue, and/or the perfusate, and/or nutrients and/or medications. These components can include, but aren't limited to including, a tissue container, pumps that enable the flow of perfusate, medications, and nutrition, tubing, sensors, and sampling/assessments containers. In an aspect, disposable assembly 113A includes disposable subassembly 303, pneumatic infusion assembly 305, perfusion pump assembly 307, tissue container assembly 309, tubing 311, output assembly 313, sensors 315, and sampler 317. In an aspect, pneumatic infusion assembly 305 drives perfusion pump assembly 307, to deliver medications and nutrition to the perfusate. In an aspect, nutrition can be delivered by one pneumatic infusion pump while medication can be delivered by another pneumatic infusion pump. In an aspect, the pneumatic infusion pumps are controlled by a controller. In an aspect, medications and/or nutrition can be delivered by a stand-alone, remotely-operated pump that is not associated with the pneumatic system. Pump choices depend upon the desired delivery rate and other factors associated with the delivered product. In an aspect, the infusion pump can include a cassette pump designed to deliver infusion materials according to desired flow rates and pressures. Perfusion pump assembly 307 includes at least one perfusion pump. In an aspect, the perfusion pump enables the flow of perfusate to and through the tissue. In an aspect, the perfusion pump can include one or more cassette pumps. Other types of pumps are contemplated by the present teachings. In an aspect, tissue container assembly 309 includes a removable tissue platform isolating the tissue from a reservoir of perfusate. In an aspect, the removable tissue platform enables coupling of the tissue with input and output fluid paths. The tissue platform enables initial cannulation of the tissue, as the cannulation can be done away from the perfusion system, and then the tissue and platform are brought to the perfusion system and simply plugged in. The tissue platform enables positioning and securing the tissue by removable fittings. In this way, de-cannulating is not required when the tissue is ready to be removed for transplant, the tissue platform fittings can simply be decoupled from the tissue container. Tissue container assembly 309 includes sample ports and various disposable sensors. Tissue container assembly 309 includes a perfusate reservoir. In an aspect, the reservoir is replenished by output from the tissue, nutrients, and medications. Replenishment occurs based on the volume of output discarded, if any. Tubing 311 connects the various parts of disposable assembly 113A to each other through tubes and appropriate connectors to form a closed loop and avoid environmental contamination. Output assembly 313 includes at least one vial to hold the output as it is measured and evaluated as described herein. The vial can be transparent for visual evaluation of the output. Disposable sensors 315 can include sensors that come into contact with the tissue and/or the perfusate. Sampler 317 receives output from the tissue to make the output available for online or offline sampling. Disposable subassembly 303 includes components such as, for example, but not limited to, an oxygenator, a hood mount, and a tissue strap. The oxygenator provides oxygen to the perfusate. The hood mount provides a surface for an environmental barrier to be coupled with the tissue container. The tissue strap maintains the position of the tissue on the tissue platform.

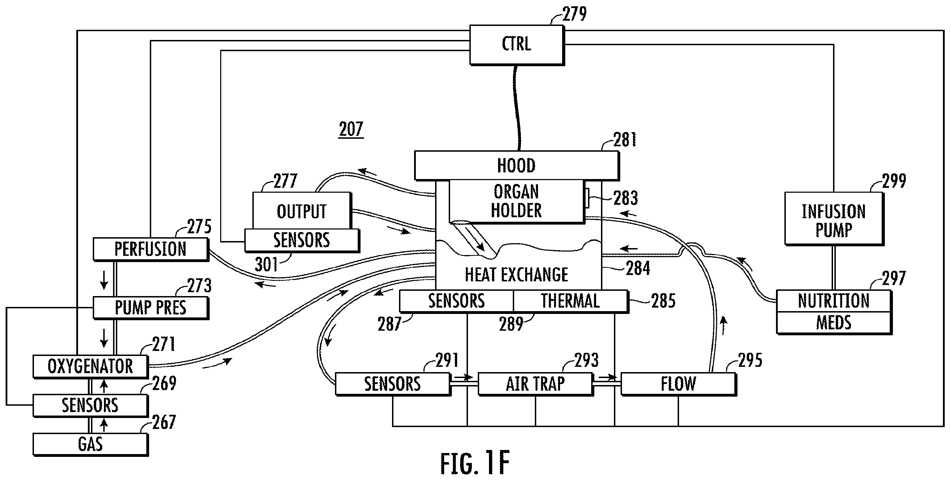

Referring now to FIG. 1F, the flow and date/control/electrical connections of an exemplary configuration of the system of the present teachings is shown. In an aspect, controller 279 controls the sequencing of events that move the perfusate, medications, and nutrition from point to point. Starting with perfusate reservoir 284, perfusate flows into and through perfusion pump 275. The pressure of the perfusate is then measured inline by pump pressure sensor 273 before the perfusate enters oxygenator 271. Oxygenated perfusate flows into heat exchanger 285 in which the perfusate temperature is adjusted to a desired level and then is assessed by inline sensors 291. Bubbles are removed by air trap 293 and inline flow rate is measured by flow meter 295. Oxygenated, de-bubbled, and thermally-adjusted perfusate is pumped into the tissue in tissue holder 283 through connectors to which the tissue is attached. The tissue processes the perfusate by producing output. Some of the output exits the tissue by orifices in the tissue itself, for example, the ureter in a kidney, and some fluid becomes available based on the process. The output that exits the tissue through a tissue orifice is pumped to an output assembly as described herein. In an aspect, the other excreted fluid follows a fluid ramp into reservoir 284. The fluid ramp enables a gentle landing of the excreted fluid into reservoir 284 to avoid damage to the contents of the perfusate. The loop continues with the perfusate pumped into perfusion pump 275. In an aspect, controller 279 tracks output that is not returned to reservoir 284. An equal amount of perfusate, and nutrition/medications 297 can be added to reservoir 284 by the pumping action of infusion pump 299. In an aspect, the system includes multiple various kinds of infusion pumps, some specific for medication delivery, some specific for nutrition delivery. In an aspect, controller 279 receives data from sensors 287 and activates thermal adjustment 289 based on the data. In an aspect, controller 279 receives data from sensors 291, air trap 293, and flow meter 295, and adjusts the characteristics, flow rate, and possibly flow volume based on those data. In an aspect, an operator can perform manual inspection of data from the sensors and can adjust, for example, but not limited to, medications, nutrition, temperature, flow rate, oxygenation, and flow volume in the perfusate to maintain the viability of the tissue. Sensors collect data about, for example, but not limited to, glucose, dissolved oxygen, temperature, pH, oxygen saturation.

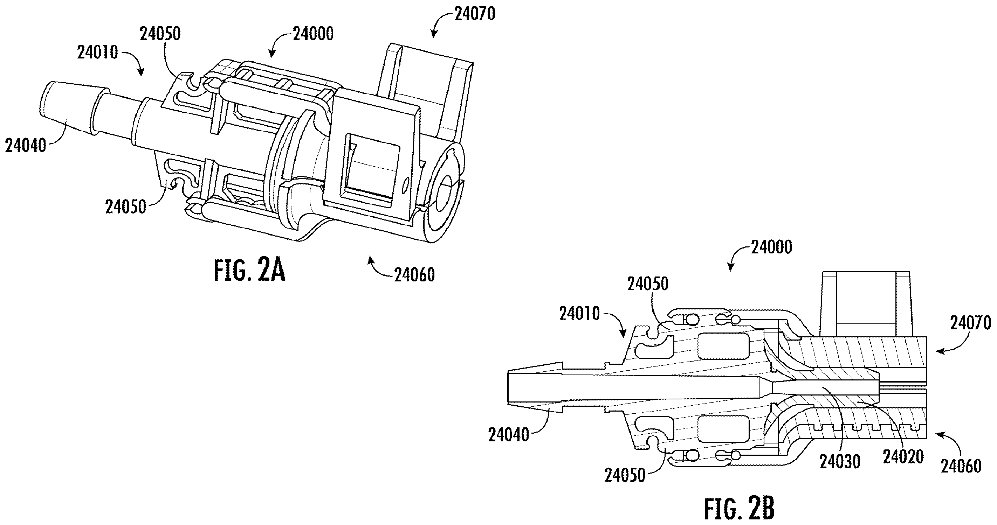

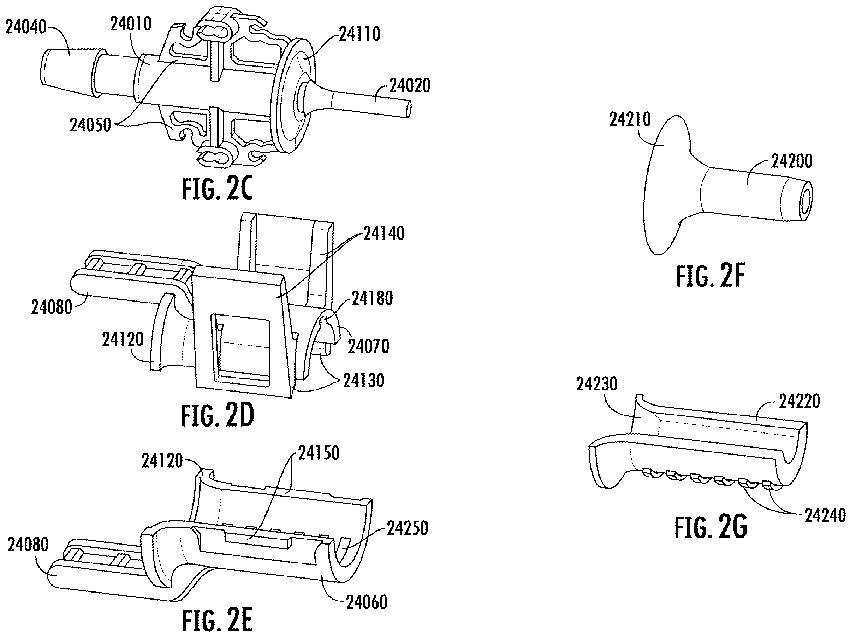

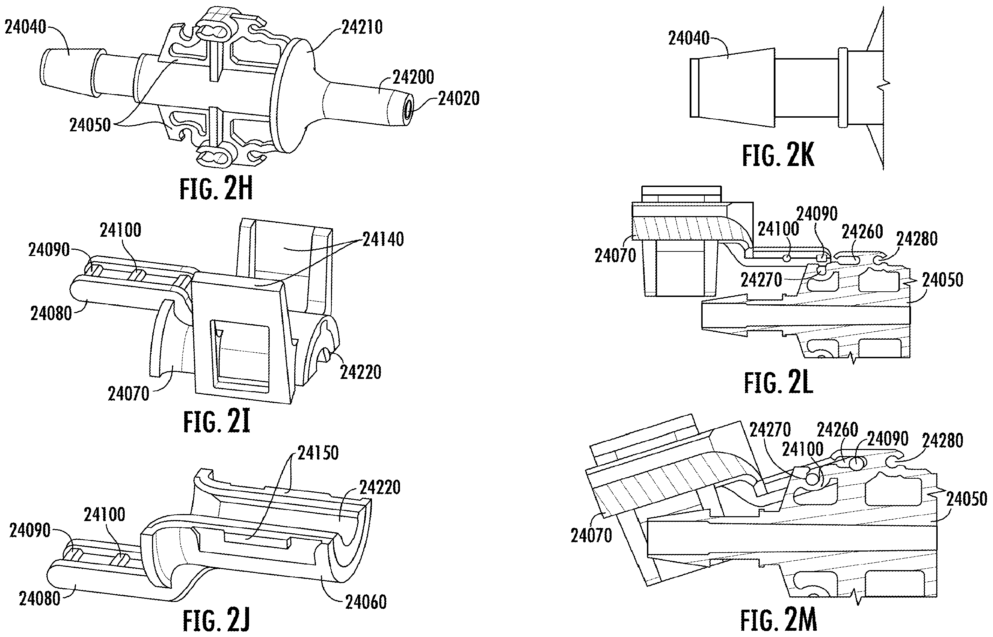

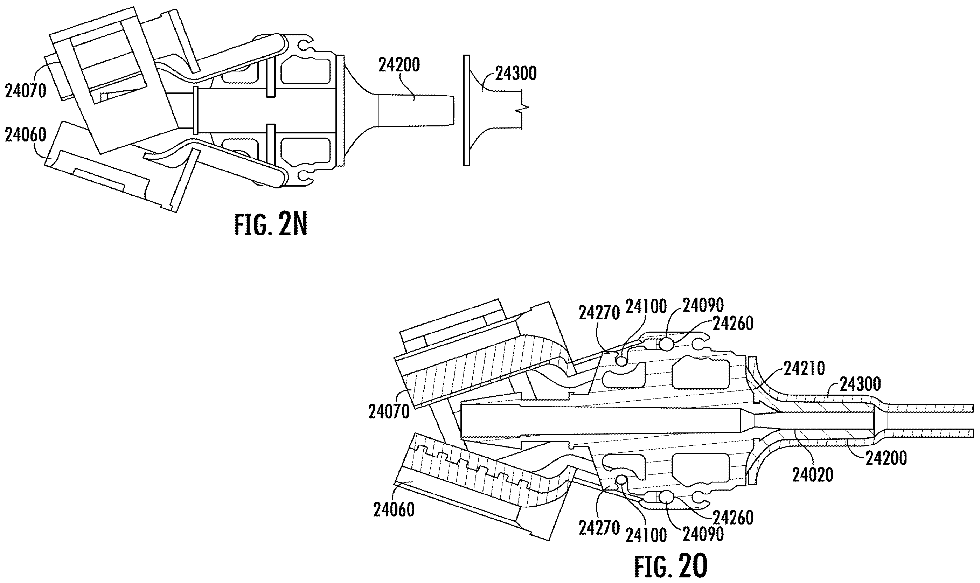

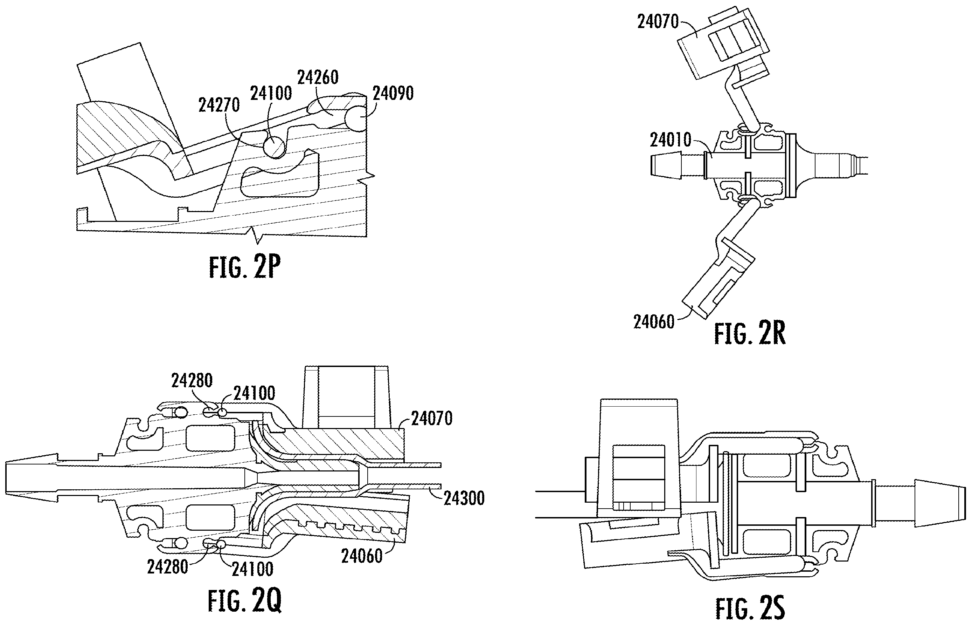

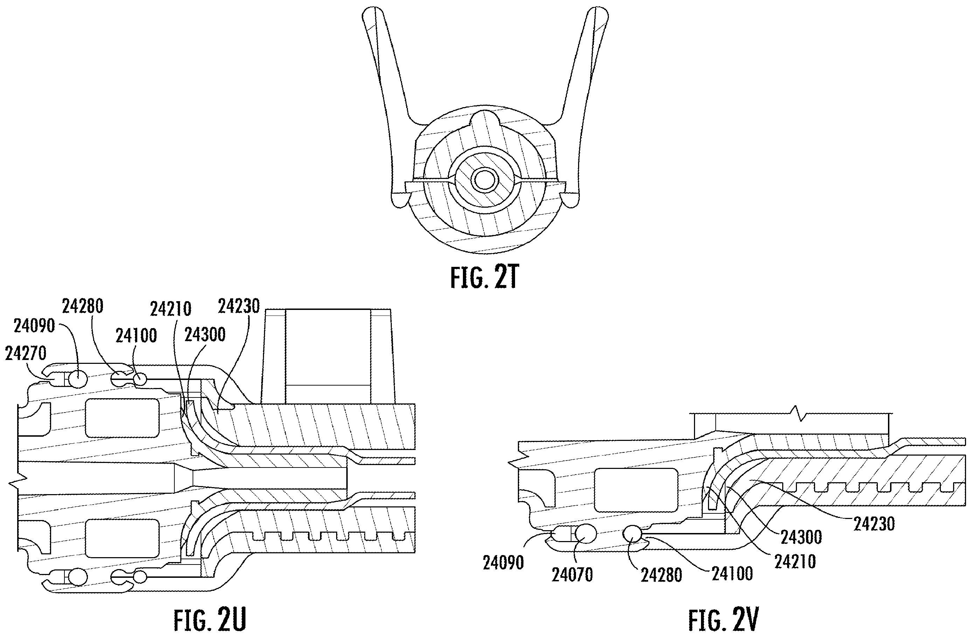

In one aspect, as seen in Figs. 2A-2X, the means for coupling to the tissue orifices includes a cannula 24000 configured and arranged at one end to interface with the tissue and at an opposing end to interface with the tubing of the perfusion loop. Generally, at Figs. 2A through 2X, the cannula 24000 includes a cannula body 24010 with at least one cannula post 24020 at one end thereof with a lumen 24030 extending there through such that the cannula post 24020 is inserted into a vessel of the tissue. For example, if configured for a kidney, the cannula post can be inserted into the artery, vein or urethra of the kidney. At the end of the cannula body 24010 opposite the cannula post 24020 is a tubing interface 24040 configured to interface with the tubing of the perfusion loop. The tubing interface 24040 may be a luer connector, barbed connector, locking connector, spin lock type connector, or any other type tubing interface connection known in the art. The cannula body 24010 further includes opposing shoulders 24050 extending outwardly from the sides of the cannula body 24010, the shoulders 24050 being configured and arranged to support and retain opposing clamping arms 24060 and 24070.

The opposing clamping arms 24060 and 24070 include pivot supports 24080 that extend rearwardly therefrom and each of the two pivot supports 24080 have a pivot bar 24090 and lock bar 24100 that are configured and arranged to engage with mating formations on the shoulders 24050 of the cannula body 24010 as will be described in detail below. The cannula post 24020 may include a flared base 24110 where it meets the cannula body 24010 and the opposing clamping arms 24060 and 24070 may include a corresponding flare 24120 at a rear portion thereof. Clamping arm 24070 includes clamp members 24130 along an edge thereof where clamp arm 24070 meets with 24060 when engaged therewith. Release tabs 24140 extend upwardly along the sides of clamping arm 24070 such that inward pressure is applied to release tabs 24140 they cause clamp members 24130 to move apart from one another to allow clamping arm 24070 to be disengaged from clamping arm 24060. Clamping arm 24070 may include a cutout region 24180 therein that allows the clamping arm to have a flexible region that allows it to deflect when pressure is applied to the release tabs 24140. Clamping arm 24060 can be seen to include clamping detents 24150 along an edge thereof where clamp arm 24060 meets with 24070 when engaged therewith. When clamping arms 24060 and 24070 are moved into engagement, clamping detents 24150 are captured and retained by clamp members 24130 to hold clamping arms 24060 and 24070 in engagement with one another about the cannula post 24020. Applying pressure to release tabs 24140 disengages clamp members 24130 from clamping detents 24150 allowing clamping arms 24060 and 24070 to be separated from one another.

Figs. 2F and 2G illustrate elastomeric components that may be included to both soften the contact regions of the cannula and provide positive sealing as between the vessel of the tissue and the cannula. A first elastomeric component 24200 is configured and arranged to have an opening there through that is received about the cannula post 24020 and includes a flared base 24210 that corresponds to the flare 24110 at the base of the cannula post 24020. A second elastomeric component comprises a pair of elastomeric components 24220 that are received on an interior surface of each of clamping arms 24060 and 24070 and include a flared base 24030 that corresponds to the flared base 24120 of clamping arms 24060 and 24070. The second elastomeric component 24220 may include engagement members 24240 that engage with slots 24250 formed in clamping arms 24060 and 24070 to assist in retaining the second elastomeric components 24220 in clamping arms 24060 and 24070. The first and second elastomeric components 24200 and 24220 may be formed from any suitable elastomeric material. Preferably, the first and second elastomeric components 24200 and 24220 are molded from silicone. More preferably, the first and second elastomeric components 24200 and 24220 are molded from silicone of 10 Shore A hardness. This is a soft silicone that prevents damage to the artery, and provides a specific compression. This may change with the clearance depending on design requirements. Further, the first and second elastomeric components 24200 and 24220 may be over molded in place or molded separately and then inserted. Preferably, when the elastomeric components 24200 and 24220 are received about the cannula post 24020 and in clamping arms 24060 and 24070, and the clamping arms 24060 and 24070 are in a closed position there is a small gap between the first and second elastomeric components. More preferably, there is between 0.1 - 0.2 mm of clearance between elastomeric components 24200 and 24220. More preferably there is about 0.15 mm of clearance between elastomeric components 24200 and 24220. The clearance allows a vessel of the tissue to be received and firmly clamped at a desired compression while also limiting damage to the vessel.

Turning now to Figs. 2H through 2M the shoulders 24050 of the cannula body 24010 can each be seen to include a pivot slot 24260, a rearward locking detent 24270, and a forward locking detent 24280. Pivot bar 24090 is received in pivot slot 24260 and allows both rotational movement of clamping arms 24060 and 24070 and a lateral sliding movement of clamping arms 24060 and 24070 forwardly and rearwardly parallel to a central axis of the cannula post 24020. Locking bar 24100 can be releasably engaged with the rearward locking detent 24270 when clamping arms 24060 and 24070 are in the open position and pivoted rearwardly.

Figs. 2N through 2V show operative modes of the cannula 24000 of the disclosure. A free end of a vessel 24300 of the tissue is received onto the first elastomeric component 24200 and cannula post 24020 and slid down to rest against the flared base 24210 while the clamping arms 24060 and 24070 are in the open position and pivoted rearwardly. The clamping arms 24060 and 24070 are then pivoted to a forward, closed position where the locking bar 24100 is in a position adjacent the front of the forward locking detent 24280. Clamping arms 24060 and 24070 are brought together and compressed so that the clamp members 24130 engage the clamping detents 24150 thereby compressing the second elastomeric components 24220 against an outer surface of the vessel 24300 which in turn compresses an inner surface of the vessel against the first elastomeric component 24200 and the cannula post 24020. Clamping arms 24060 and 24070, now in the closed, latched position are slid rearwardly relative to the cannula body 24010 such that the flared end 24230 contacts and compresses the vessel 24300 against the flared base 24210 to assist in sealing and retaining the vessel relative to the cannula. As the clamping arms 24060 and 24070 slide rearwardly, the locking bar 24100 enters and engages with the locking detent 24280 and the pivot bar 24090 slides into the pivot slot 24070 such that flat sides on the pivot bar 24090 engage with flat walls on the pivot slot 24070. In this manner, the pivot bar 24090, the locking bar 24100 and the engaged clamping arms 24060 and 24070 cooperate to maintain the cannula in a clamped, sealed position about the vessel 24300.

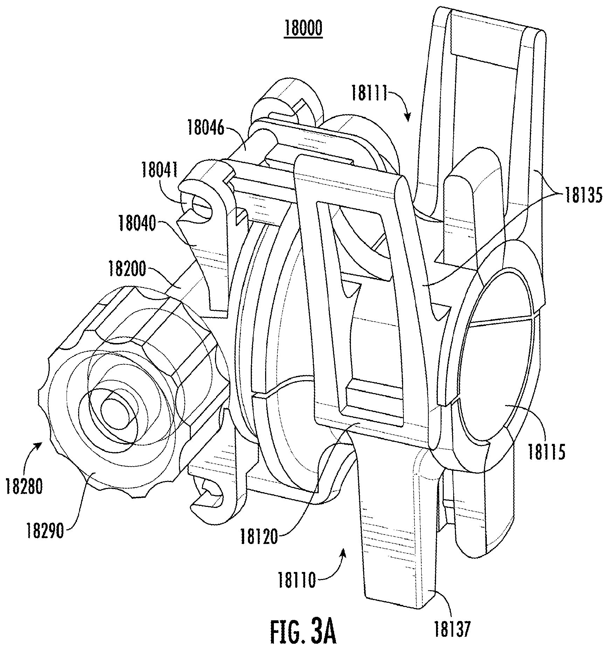

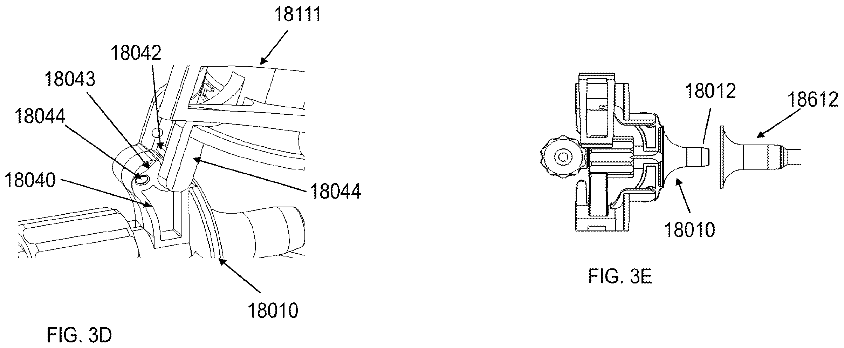

Referring now to Figs. 3A-3E, in one aspect, the means for coupling to the tissue orifices includes a cannula 18000 configured and arranged at one end to interface with the tissue, at another end to interface with the tubing of the perfusion loop, and at a third end to optionally interface with additional cannula 18000. Generally, at Figs. 3A-3E, the cannula 18000 includes a cannula body 18010 with at least one cannula post 18012 at one end thereof, the cannula post 18012 can be inserted into the artery, vein, or urethra of the kidney. Connected to the cannula body 18010 by way of a tee fitting interface 18275 located on a first end of the cannula body 18010 is a tee fitting 18200. The tee fitting 18200 can have three interfaces; a perfusion loop interface 18260 configured to interface with the tubing of the perfusion loop; a primary cannula interface 18270 configured to interface with cannula body 18010; and a secondary cannula interface 18280 configured to be interface with another cannula 18000 or to be capped. The three interfaces of the tee fitting 18200 interface with their respective components through a plurality of connector fittings 18290. The plurality of connector fittings 18290 may be a luer connector, luer cap screws, barbed connector, locking connector, spin lock type connector, or any other type of interface connection known in the art.

The opposing clamping arms 18110 and 18111 include pivot supports 18045 that extend rearwardly therefrom and have a pivot bar 18046 that are configured to engage with pivot slots 18041 located on the end of shoulders 18040 that extend from the cannula body 18010. The cannula post 18012 may include a flared base 18050 where it meets the cannula body 18010 and the opposing clamping arms 18110 and 18111 may include a corresponding flared shape. Clamping arm 18111 includes clamp members 18120 along an edge thereof where clamping arm 18111 meets with clamping arm 18110. Clamping arm 18110 can be seen to include clamping detents 18125 along an edge thereof where clamping arm 18110 meets with clamping arm 18111 when engaged therewith. When clamping arms 18110 and 18111 are moved into engagement, clamping detents 18125 are captured and retained by clamp members 18120 to hold clamping arms 18110 and 18111 in engagement with one another about the cannula post 18012. Clamping arm 18110 has collar posts 18137 extending outward, and clamping are 18111 has release tabs extending outward. The collar posts 18137 have collar post detents 18138 on the inside surface that are configured to interface and lock with a tray 18537 Applying pressure to release tabs 18135 disengages clamp members 18120 from clamping detents 18125 allowing clamping arms 18110 and 18111 to be separated from one another. The cannula may be configured to be between 3 and 9 mm.

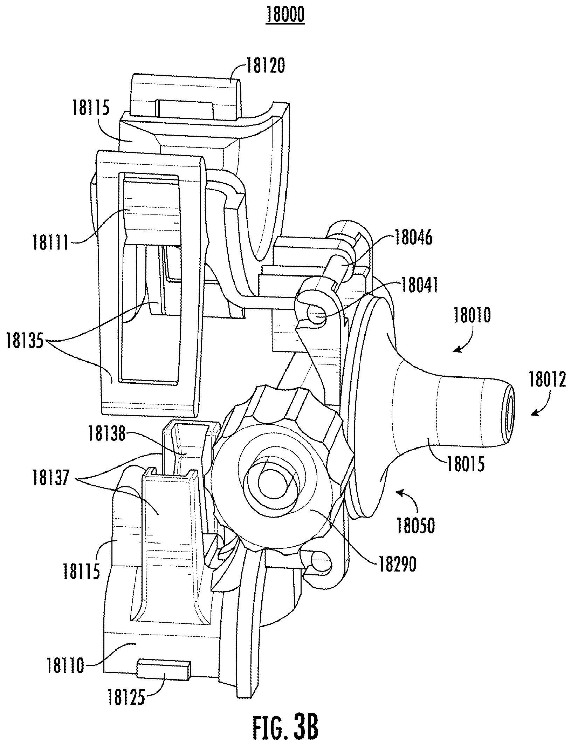

Fig. 3B illustrates elastomeric components 18015 and 18115 that may be included to both soften the contact regions of the cannula 18000 and provide positive sealing as between the vessel of the tissue and the cannula 18000. A first elastomeric component 18015 is configures and arranged to have an opening there through that is received about the cannula post 18012 and includes a flare that corresponds to the flared base 18050 of the cannula post 18012. A second elastomeric component 18115 is received by each clamping arm 18110 and 18111, and have a flare that corresponds to the flared base 18050 of the cannula post 18012. The first and second elastomeric components 18015 and 18115 may be formed from any suitable elastomeric material. Preferably, the first and second elastomeric components 18015 and 18115 are molded from silicone. More preferably, the first and second elastomeric components 18015 and 18115 are molded from silicone of 10 Shore A hardness. This is a soft silicone that prevents damage to the artery, and provides a specific compression. This may change with the clearance depending on design requirements. Further, the first and second elastomeric components 18015 and 18115 may be molded in place or molded separately and then inserted. Preferably, when the elastomeric components 18015 and 18115 are received about the cannula post 18012 and in clamping arms 18110 and 18111, and the clamping arms 18110 and 18111 are in a closed position there is a small gap between the first and second elastomeric components 18015 and 18115. More preferably there is about 0.1 - 0.2 mm of clearance between elastomeric components 18115 and 18151. More preferably there is about 0.15 mm of clearance between elastomeric components 18015 and 18115. The clearance allows a vessel of the tissue to be received and firmly clamped at a desired compression while also limiting damage to the vessel.

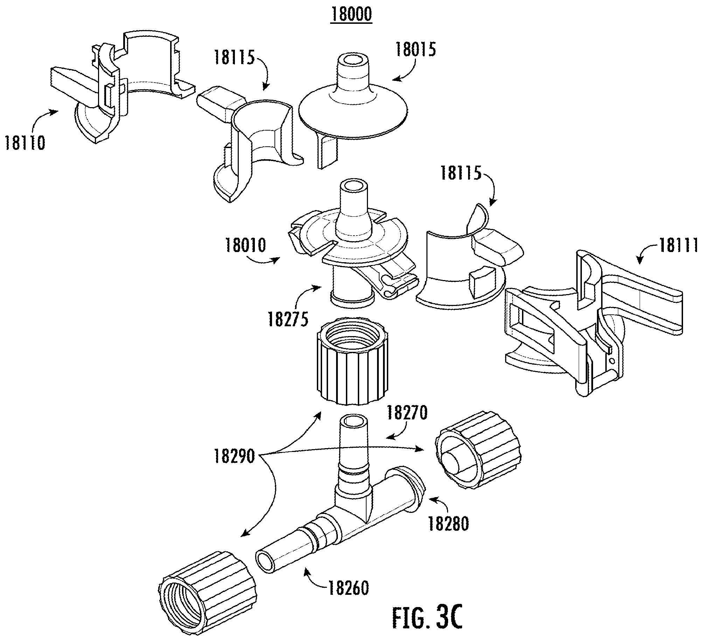

Referring now to Fig. 3C, which illustrates an exploded view of an exemplary cannula 18000. From this exploded view, the contents of the cannula 18000 is made clear. In some aspects, the cannula body 18010 is configured to receive a first elastomeric component 18015 which can be pulled over the cannula body 18015. The clamping arms 18110 and 18111 are similarly configured to receive a second elastomeric component 18115. Once the clamping arms 18110 and 18111 and cannula body 18010 are fitted with their respective elastomeric components 18015 and 18115 the clamping arms 18110 and 18110 can be connected to the cannula body 18010. This is achieved by snapping the pivot bar 18046 into the pivot slot 18041. The tee fitting 18260, has three interfaces; a perfusion loop interface 18260, a primary cannula interface 18270 and a secondary cannula interface 18280. The cannula body 18010 also has an interface, a tee fitting interface 18275. Each of the interfaces is configured to interact with a connector fitting 18290 to interface with its respective counterpart. For example, a threaded luer fitting may be used to interface the primary cannula interface 18270 with the tee fitting interface 18275 of the cannula body 18010. Another example is that the secondary cannula interface 18280 may be fitted with a luer cap screw, or it may be fitted with a threaded luer fitting to connect to the perfusion loop interface of another cannula.

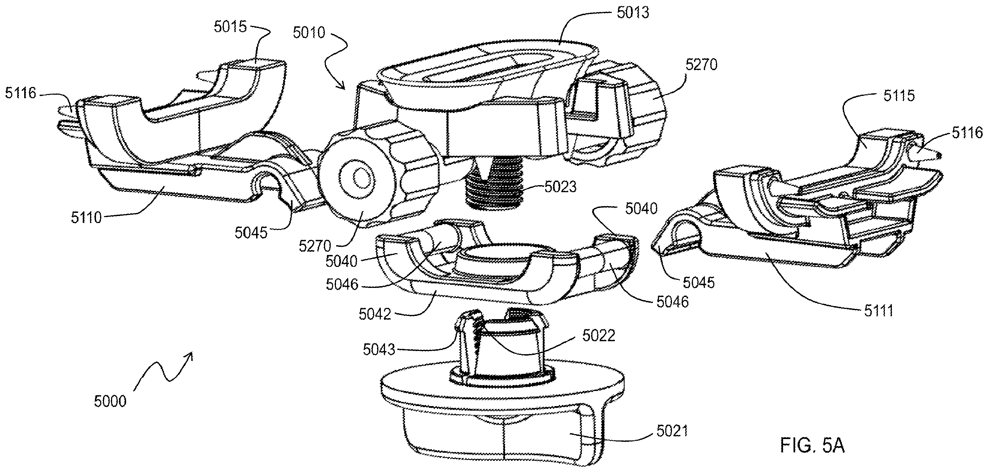

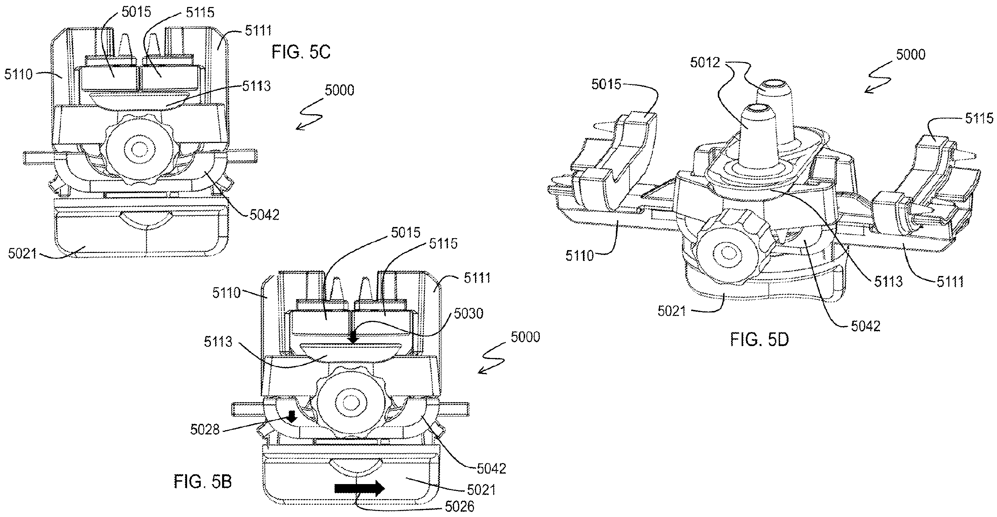

Referring now to Figs. 3D-3E, the cannula body 18010 can be seen to include a shoulder 18040, a pivot slot 18041, a detent guide 18043 and a cannula body pocket 18044. When clamping arms 18110 and 18111 are not engaged with one another and the cannula 18000 is moving to engage with a tissue, the clamping arms 18110 and 18111 are configured to rotate backwards by the pivot bars 18046 that are engaged with the pivot slots 18041. Rotating the clamping arms 18110 and 18111 backwards causes collar detents 18042 that are on the inside of the pivot supports 18045 to follow a detent guide 18043 near the pivot slot 18041 and lock into a cannula body pocket 18044. The cannula body pocket 18044 allows the clamping arms 18110 and 18110 to be secured in an open position without having to lock them as described in Figs. 5A-5X.

Similarly, as described above with respect to the previous embodiment. operative modes of the cannula 18000 of the disclosure include a free end of a tissue vessel 18612 is received onto the first elastomeric component 18015 and cannula post 18012 and is slid down to rest against the flared base 18050 of the cannula post 18012 while the clamping arms 18110 and 18111 are locked into the cannula body pockets 18044. The clamping arms 18110 and 18111 are then pivoted to a forward, closed position where the clamp members 18120 engage with the clamping detents 18125 thereby compressing the second elastomeric components 18115 against the outer surface of the tissue vessel 18613 which in turn compresses the inner surface of the tissue vessel 18613 against the first elastomeric component 18015 and the cannula post 18012. With the clamping arms 18110 and 18111 in the closed, latched position the tissue vessel 18613 is compressed to assist in sealing and retaining the tissue vessel 18613 relative to the cannula 18000.

Perfusate may flow in through the perfusion loop interface 18260 and then upwards through the primary cannula interface 18270 and through the cannula body 18010 and into the tissue in which the cannula 1800 is connected to. Perfusate may also flow in through the perfusion loop interface 18260 and then through the secondary cannula interface 18280 and into a secondary cannula that is attached to the first cannula 18000 by means of the secondary cannula interface. Alternatively, the secondary cannula interface 18280 may be capped and perfusate flow will flow only in through the perfusion loop interface 18260 and into the one cannula 18000 connected to the tee fitting 18200. The design of the tee fitting 18200 allows for a more compactly sized cannula 18000 compared to the cannula described in Figs. 2A-2V.

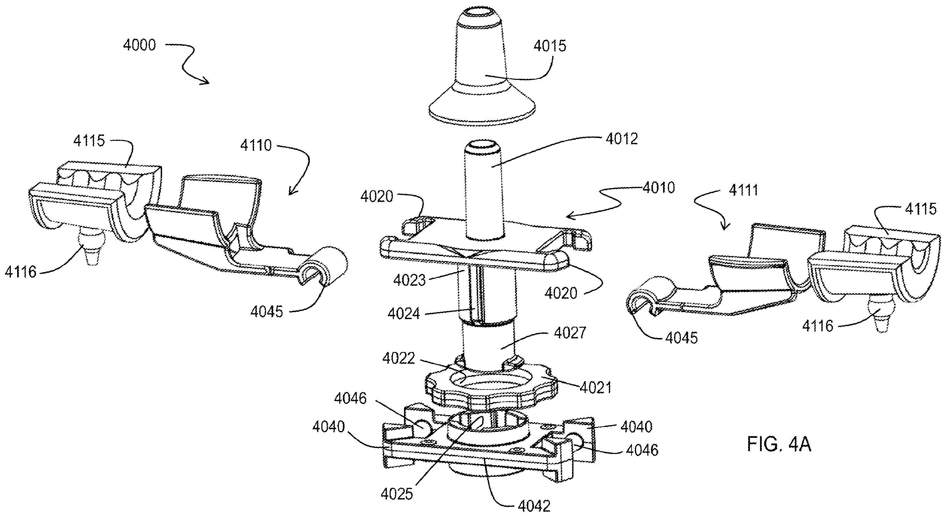

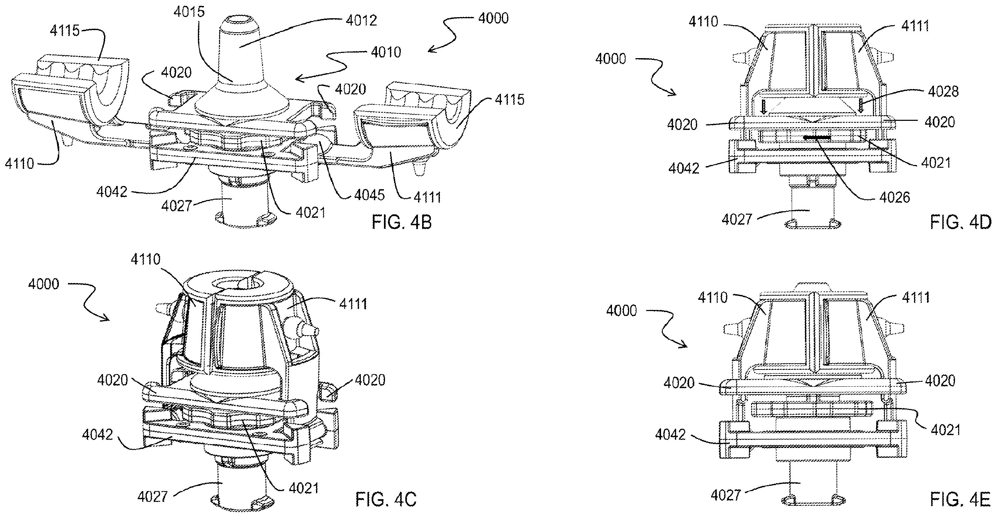

Referring now to Figs. 4A-4E, in one aspect, the means for coupling to the tissue orifices includes a cannula 4000 configured and arranged at one end to interface with the tissue, at another end to interface with the tubing of the perfusion loop. Generally, at Figs. 4A-4E, the cannula 4000 includes a cannula body 4010 with at least one cannula post 4012 at one end thereof, the cannula post 4012 can be inserted into the artery, vein, or urethra of the kidney. At the opposing end of the cannula body 4010 is a cannula interface 4270 allowing the cannula 4000 to be connected to the perfusion loop. The cannula interface 4270 may be configured for interface with a plurality of connector fittings including, but not limited to a luer connector, luer cap screws, barbed connector, locking connector, spin lock type connector, or any other type of interface connection known in the art.

Opposing clamping arms 4110 and 4111 include pivot supports 4045 that extend rearwardly therefrom. Pivot bars 4046, configured to engage with pivot supports 4045 are located on in end of shoulders 4040 that extend from a clamp support platform 4042. Clamp members 4120 extend outwardly from the cannula body 4010 and have slots therein to hold clamping arms 4110 and 4111 in a closed, clamped position when the clamping arms 4110 and 4111 are rotated into an upward position against the cannula post 4012. When clamping arms 4110 and 4111 are moved into engagement, clamping arms 4110 and 4111 are captured and retained by clamp members 4120 to hold clamping arms 4110 and 4111 in engagement with one another about the cannula post 4012. The cannula body may be configured to have a diameter of between 3 and 9 mm.

Figs. 4A and 4B illustrate elastomeric components 4015 and 4115 that may be included to both soften the contact regions of the cannula 4000 and provide positive sealing as between the vessel of the tissue and the cannula 4000. A first elastomeric component 4015 is configured and arranged to have an opening there through that is received about the cannula post 4012 and includes a flare that corresponds to the flared base of the cannula post 4012. A second elastomeric component 4115 is received by each clamping arm 4110 and 4111. The first and second elastomeric components 4015 and 4115 may be formed from any suitable elastomeric material. Preferably, the first and second elastomeric components 4015 and 4115 are molded from silicone. More preferably, the first and second elastomeric components 4015 and 4115 are molded from silicone of 10 Shore A hardness. This is a soft silicone that prevents damage to the artery, and provides a specific compression. This may change with the clearance depending on design requirements. Further, the first and second elastomeric components 4015 and 4115 may be molded in place or molded separately and then inserted. A detent 4116 may be provided on elastomeric components 4115 to be received into a corresponding opening in clamping arms 4110 and 4111 to assist in maintaining them in their installed position. Preferably, when the elastomeric components 4015 and 4115 are received about the cannula post 4012 and in clamping arms 4110 and 4111, and the clamping arms 4110 and 4111 are in a closed position there is a small gap between the first and second elastomeric components 4015 and 4115. More preferably there is about 0.1 - 0.2 mm of clearance between elastomeric components 4015 and 4115. More preferably there is about 0.15 mm of clearance between elastomeric components 4015 and 4115. The clearance allows a vessel of the tissue to be received and firmly clamped at a desired compression while also limiting damage to the vessel.

Referring back to Fig. 4A, which illustrates an exploded view of an exemplary cannula 4000. From this exploded view, the elements of the cannula 4000 are made clear. In some aspects, the cannula body 4010 is configured to receive a first elastomeric component 4015 which can be pulled over the cannula post 4012. The clamping arms 4110 and 4111 are similarly configured to receive a second elastomeric component 4115. Once the clamping arms 4110 and 4111 and cannula body 4010 are fitted with their respective elastomeric components 4015 and 4115 the clamping arms 4110 and 4110 can be connected to the cannula body 4010. This is achieved by snapping the pivot bar 4046 into the pivot slot 4045.

Referring now to Figs. 4B-4C, as described above with respect to the previous embodiment. operative modes of the cannula 4000 are disclosed wherein a free end of a tissue vessel is received onto the first elastomeric component 4015 and cannula post 4012 and is slid down to rest against the flared base of the cannula post 4012 while the clamping arms 4110 and 4111 are rotated into an open position. As seen at Fig. 4C the clamping arms 4110 and 4111 are then pivoted to a forward, closed position where the clamp members 4040 engage with clamping arms 4110 and 4111 thereby compressing the second elastomeric components 4115 against the outer surface of the tissue vessel which in turn compresses the inner surface of the tissue vessel against the first elastomeric component 4015 and the cannula post 4012.

With the clamping arms 4110 and 4111 in the closed, latched position clamping dial 4021 seen received about the cannula body 4010 is rotatable and is displaced in a linear manner by mating thread formations 4022 and 4023 on an interior of clamping dial 4021 and about the cannula body 4010 respectively. As best seen in Fig. 4D, rotation in the direction of arrow 4026 (rotation in either direction is possible depending on whether thread formations 4022 and 4023 are formed as right or left handed threads) caused linear displacement of clamping dial 4021 relative to the cannula body 4010. Linear displacement of the clamping dial 4021 exerts pressure on clamp support platform 4042 displacing it in the direction of arrows 4028 in turn causing displacement of the clamping arms 4110 and 4111 in order to clamp the bottom of the clamping arms 4110 and 4111 downwardly against the flared base of the cannula post 4012 and compressing the tissue vessel to assist in sealing and retaining the tissue vessel relative to the cannula 4000 as seen at Fig, 4E. Rotation of the clamping dial 4021 in the opposite direction reversed the displacement and allows release of the compression of the tissue vessel. Key 4024 positioned on cannula body 4010 is slidably received in keyway 4025 to prevent rotation of clamp support platform 4042 relative to the cannula body 4010 during rotation of the clamping dial 4021 to ensure smooth linear displacement of the clamp support platform 4042.

Referring now to Figs. 5A-5D, in one aspect, the means for coupling to the tissue orifices includes a cannula 5000 configured and arranged at one end to interface with the tissue, at another end to interface with the tubing of the perfusion loop. Generally, the cannula 5000 includes a cannula body 5010 with at least one tissue interface pad 5013 at one end thereof. The tissue interface pad 5013 is shown to have a configuration that is elongated to accept the terminal end of one or more vessels extending from a tissue to be perfused. At the opposing end of the cannula body 5010 is a cannula interface 5270 allowing the cannula 5000 to be connected to the perfusion loop. The cannula interface 5270 is shown hear as having two ends that may be configured for interface with a plurality of connector fittings including, but not limited to a luer connector, luer cap screws, barbed connector, locking connector, spin lock type connector, or any other type of interface connection known in the art. One shilled in the art should appreciate that the cannula interface 5270 may also be singular as shown in other embodiments illustrated herein.

Opposing clamping arms 5110 and 5111 include pivot supports 5045 that extend rearwardly therefrom. Pivot bars 5046, configured to engage with pivot supports 5045 are located on an end of shoulders 5040 that extend from a clamp support platform 5042.

Tissue interface pad 5013 may be formed as an elastomeric component. Further elastomeric components 5015 and 5115 that may be included to both soften the contact regions of the cannula 5000 and provide positive sealing as between the vessel of the tissue and the cannula 5000. First and second elastomeric components 5015 and 5115 are received by each clamping arm 5110 and 5111. The elastomeric components may be formed from any suitable elastomeric material. Preferably, the elastomeric components are molded from silicone. More preferably, the elastomeric components are molded from silicone of 10 Shore A hardness. This is a soft silicone that prevents damage to the artery, and provides a specific compression. This may change with the clearance depending on design requirements. Further, the elastomeric components may be molded in place or molded separately and then inserted. Detents 5116 may be provided on elastomeric components to be received into a corresponding opening in clamping arms 5110 and 5111 to assist in maintaining them in their installed position.

Referring to Fig. 5D, in some aspects, the cannula body 5010 is configured to receive one or more cannula posts 5012 against the tissue support pad 5013 wherein an additional elastomeric component can be pulled over the cannula post 5012. The inclusion of cannula posts 5012 in this configuration is optional as they are not required when clamping to the terminal end of a vessel. The clamping arms 5110 and 5111 are similarly configured to receive elastomeric components 5115. Preferably, when the elastomeric components 4013 are received about the cannula post 5012 and in clamping arms 5110 and 5111, and the clamping arms 5110 and 5111 are in a closed position there is a small gap between the first and second elastomeric components 5115 and the optional cannula post 5012. More preferably there is about 0.1 - 0.2 mm of clearance between elastomeric components 5115 and the optional cannula post 5012. More preferably there is about 0.15 mm of clearance between elastomeric components 5115 and the optional cannula post 5012. The clearance allows a vessel of the tissue to be received and firmly clamped at a desired compression while also limiting damage to the vessel.

Referring now to Figs. 5B-5D, as described above with respect to the previous embodiment. operative modes of the cannula 5000 are disclosed wherein a free end of a tissue vessel is received onto the tissue interface pad 5113 or onto the optional cannula post 5012 adjacent the tissue interface pad 5113 while the clamping arms 5110 and 5111 are rotated into an open position as illustrated at Fig. 5D. As seen at Fig. 5B the clamping arms 5110 and 5111 are then pivoted to a forward, closed position where the elastomeric components 5015 and 5115 within the clamping arms 5110 and 5111 compress the elastomeric components 5015 and 5115 against the outer surface of the tissue vessel which in turn, if a cannula post 5012 is being used, compresses the inner surface of the tissue vessel against the cannula post 5012.

With the clamping arms 5110 and 5111 in the closed position clamping dial 5021 seen received about the cannula body 5010 is rotatable and is displaced in a linear manner by mating thread formations 5022 and 5023 on an interior of clamping dial 5021 and about the cannula body 5010 respectively. As best seen in Fig. 5B, rotation in the direction of arrow 5026 (rotation in either direction is possible depending on whether thread formations 5022 and 5023 are formed as right or left handed threads) causes linear displacement of clamping dial 5021 relative to the cannula body 5010. Linear displacement of the clamping dial 5021 exerts pressure on clamp support platform 5042 via detents 5043 extending from fingers on the clamping dial 5021 that engage with an opening in the clamp support platform 5042 thereby displacing it in the direction of arrows 5028 in turn causing displacement of the clamping arms 5110 and 5111 in the direction of arrow 5030 in order to clamp the bottom of the clamping arms 5110 and 5111 downwardly against the tissue interface pad 5113 and compressing the tissue vessel to assist in sealing and retaining the tissue vessel relative to the cannula 5000 as seen at Fig, 5C. Rotation of the clamping dial 5021 in the opposite direction reverses the displacement and allows release of the compression of the tissue vessel.

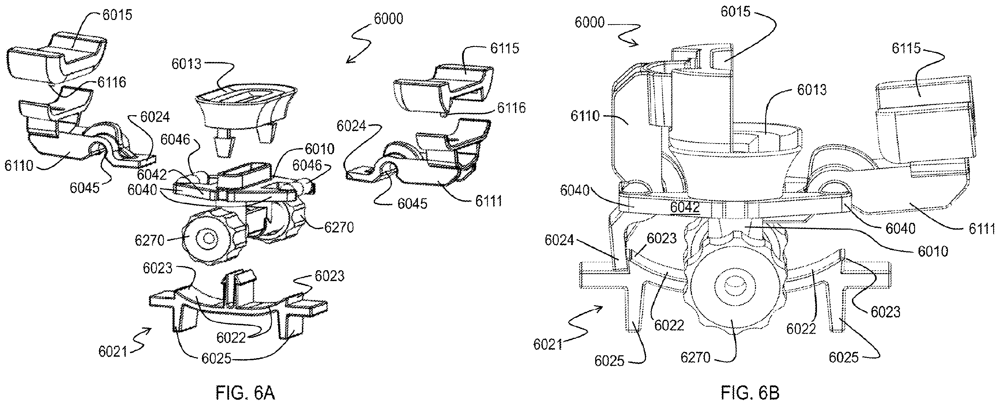

Turning to Figs. 6A-6B, in one aspect, the means for coupling to the tissue orifices includes a cannula 6000 configured and arranged at one end to interface with the tissue, at another end to interface with the tubing of the perfusion loop. Generally, the cannula 6000 includes a cannula body 6010 with at least one tissue interface pad 6013 at one end thereof. The tissue interface pad 6013 is shown to have a configuration that is elongated to accept the terminal end of one or more vessels extending from a tissue to be perfused, although the tissue interface pad 6013 may also be generally circular to interface with a singular vessel as well. At the opposing end of the cannula body 6010 is a cannula interface 6270 allowing the cannula 6000 to be connected to the perfusion loop. The cannula interface 6270 is shown hear as having two ends that may be configured for interface with a plurality of connector fittings including, but not limited to a luer connector, luer cap screws, barbed connector, locking connector, spin lock type connector, or any other type of interface connection known in the art. One skilled in the art should appreciate that the cannula interface 6270 may also be singular as shown in other embodiments illustrated herein.

Opposing clamping arms 6110 and 6111 include pivot supports 6045 that extend rearwardly therefrom. Pivot bars 6046, configured to engage with pivot supports 6045 are located on an end of shoulders 6040 that extend from a clamp support platform 6042.

Tissue interface pad 6013 may be formed as an elastomeric component. Further elastomeric components 6015 and 6115 that may be included to both soften the contact regions of the cannula 6000 and provide positive sealing as between the vessel of the tissue and the cannula 6000. First and second elastomeric components 6015 and 6115 are received by each clamping arm 6110 and 6111. The elastomeric components may be formed from any suitable elastomeric material. Preferably, the elastomeric components are molded from silicone. More preferably, the elastomeric components are molded from silicone of 10 Shore A hardness This is a soft silicone that prevents damage to the artery, and provides a specific compression. This may change with the clearance depending on design requirements. Further, the elastomeric components may be molded in place or molded separately and then inserted. Detents 6116 may be provided on elastomeric components to be received into a corresponding opening in clamping arms 6110 and 6111 to assist in maintaining them in their installed position.

As described above with respect to the previous embodiment, operative modes of the cannula 6000 are disclosed wherein a free end of a tissue vessel is received onto the tissue interface pad 6113. As seen at Fig. 6B the clamping arms 6110 and 6111 are pivoted to a forward, closed position where the elastomeric components 6015 and 6115 within the clamping arms 6110 and 6111 compress the elastomeric components 6015 and 6115 against the outer surface of the tissue vessel, which in turn compresses the terminal end of the tissue vessel against the tissue interface pad 6013.

With the clamping arms 6110 and 6111 in the closed position, clamping lock 6021 seen received into the bottom of the cannula body 6010 includes ramps 6022 and detents 6023 that serve to engage locking tabs 6024 on the ends of clamping arms 6110 and 6111. When the clamping arms 6110 and 6111 are rotated upwardly to a closed position, the locking tabs move along the ramps 6023 displacing the clamping lock 6021 downwardly until the reach the fully closed position wherein the locking tabs 6024 drop off of the ramps 6023 behind the detents 6023 thereby retaining the clamping arms 6110 and 6111 from rotating out of the closed position. To remove the terminal end of the tissue from the cannula 6000, a user can depress the release tabs 6025 causing the clamping lock 6021 ends to deflect downwardly until the detents 6023 release the locking tabs 6024 allowing the clamping arms 6110 and 6111 to be rotated downwardly to an open position.

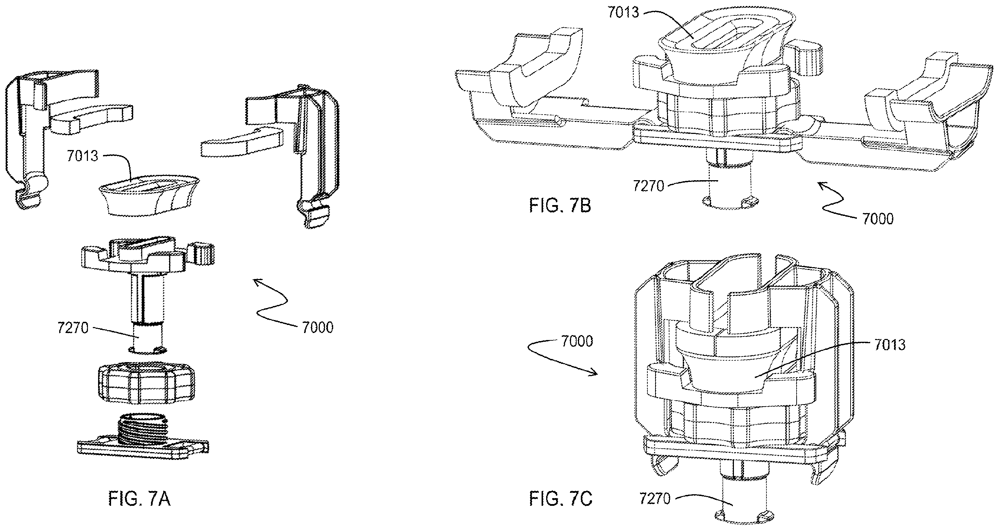

The cannula 7000 depicted in Figs. 7A-7C include many features that correspond to the above described embodiments. This arrangement is shown primarily to illustrate that an elongate tissue interface pad 7013 can be employed in conjunction with a singular cannula interface 7270. One skilled in the art should appreciate that any combination of the various features shown in the multiple embodiments disclosed herein are for illustrative purposes and the various features can be recombined in virtually any combination with one another and still fall within the scope of the present disclosure.

In all disclosed embodiments, perfusate may flow from a perfusion loop into the cannula interface and then upwards through lumen in the cannula body and if used through a lumen in the cannula post and into the tissue to which the cannula is connected. In embodiments that include a dual ended cannula interface, perfusate may flow from a perfusion loop via a first cannula interface and into both the cannula body and through the secondary cannula interface to further service a secondary cannula that is attached to the first cannula by means of the secondary cannula interface. Alternatively, the secondary cannula interface may be capped and perfusate flow will flow only in through the cannula interface and into the one cannula body connected to the tee fitting.

Referring back to Fig. 1F, starting with fluid in a reservoir (not shown) in tissue container, perfusate exits tissue container 284 in tubing and enters perfusion pump 275, and is pumped through/from perfusion pump 275. The perfusate travels past sensors 301, past pressure sensor 273 and possibly other sensors, into oxygenator 271, and into thermal exchange area 371, in the direction of arrow 203. Perfusate exits thermal exchange area 285, past sensors 291, such as a pressure sensor, and into bubble trap 293. Perfusate exits bubble trap 293 past durable flow meter 295 into the cannulated tissue through connector 283. Fluids associated with the functioning of the tissue drain into the reservoir, and the closed loop perfusate movement continues. A vent line includes a sterile filter that vents to atmosphere. In an aspect, one end of the vent line is coupled with the tissue container, and the other end is coupled with a pump that is used to set up a slightly negative pressure, just below atmospheric pressure, within the tissue container. In an aspect, a pressure in the tissue container that is slightly lower than venous pressure mimics interstitial pressure, which is slightly negative to venous pressure, encouraging integrity in the veins, and discouraging kinking and collapsing of the veins. Other flow paths are contemplated by the exemplary configuration of the present teachings. Alternative flow paths can be "manually" or automatically initiated. In an aspect, possible flow paths can be displayed at a user interface, and the user can pick a flow path. In an aspect, a controller can access a recipe and/or the user-selected flow path and open/close valves associated with the pneumatic assembly in order to move perfusate and/or infused material in paths possibly different from the depicted path.

Various alternatives and modifications can be devised by those skilled in the art without departing from the scope of the invention as defined by the appended claims. Accordingly, the present disclosure is intended to embrace all such alternatives, modifications and variances. Additionally, while several example configurations of the present disclosure have been shown in the drawings and/or discussed herein, it is not intended that the disclosure be limited thereto. Therefore, the above description should not be construed as limiting, but merely as exemplifications of particular configurations. In addition, those skilled in the art will envision other modifications within the scope of the claims appended hereto.

The drawings are presented only to demonstrate certain examples of the disclosure. And, the drawings described are only illustrative and are non-limiting. In the drawings, for illustrative purposes, the size of some of the elements may be exaggerated and not drawn to a particular scale. Additionally, elements shown within the drawings that have the same numbers may be identical elements or may be similar elements, depending on the context.

Where the term "comprising" is used in the present description and claims, it does not exclude other elements or steps. Where an indefinite or definite article is used when referring to a singular noun, e.g. "a" "an" or "the", this includes a plural of that noun unless something otherwise is specifically stated. Hence, the term "comprising" should not be interpreted as being restricted to the items listed thereafter; it does not exclude other elements or steps, and so the scope of the expression "a device comprising items A and B" should not be limited to devices consisting only of components A and B.

Furthermore, the terms "first", "second", "third," and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the example configurations of the disclosure described herein are capable of operation in other sequences and/or arrangements than are described or illustrated herein.

## Claims

1. A cannula (24000, 18000, 4000, 5000, 6000, 7000) for connecting a vessel (24300, 18613) of a tissue to be perfused to a perfusion loop, comprising:
a cannula body (24010, 18010, 4010, 5010, 6010) with a lumen (24030) extending there through;
a tubing interface (24040, 18260, 4270, 5270, 6270, 7270) at a first end of the cannula body (24010, 18010, 4010, 5010, 6010) for connection with the perfusion loop;a cannula post (24020, 18012, 4012, 5012) at a second end of the cannula body (24010, 18010, 4010, 5010, 6010) configured to receive the vessel (24300, 18613) of the tissue to be perfused; and
two clamping arms (24060, 24070, 18110, 18111, 4110, 4111, 5110, 5111, 6110, 6111) pivotally connected to said cannula body (24010, 18010, 4010, 5010, 6010) operable to retain at least one vessel (24300, 18613) of the tissue to be perfused in compression about the cannula post (24020, 18012, 4012, 5012).

2. The cannula (24000, 18000, 4000, 5000, 6000, 7000) of Claim 1, wherein said two clamping arms (24060, 24070, 18110, 18111, 4110, 4111, 5110, 5111, 6110, 6111) have an open position wherein the clamping arms (24060, 24070, 18110, 18111, 4110, 4111, 5110, 5111, 6110, 6111) are pivoted rearward relative to said cannula body (24010, 18010, 4010, 5010, 6010) and a clamped position wherein the clamping arms (24060, 24070, 18110, 18111, 4110, 4111, 5110, 5111, 6110, 6111) pivoted forward relative to said cannula body (24010, 18010, 4010, 5010, 6010) and are engaged with one another about said vessel (24300, 18613) of the tissue to be perfused and the cannula post (24020, 18012, 4012, 5012).

3. The cannula (24000, 18000, 4000, 5000, 6000, 7000) of Claim 1 or Claim 2, further comprising:
a first elastomeric component (24200, 18015, 4015, 5015, 6015) positioned about said cannula post (24020, 18012, 4012, 5012); and
a second elastomeric component (24220, 18115, 4115, 5115, 6115) positioned within each of said clamping arms (24060, 24070, 18110, 18111, 4110, 4111, 5110, 5111, 6110, 6111).

4. The cannula (24000, 18000, 4000, 5000, 6000, 7000) of Claim 3, wherein said first elastomeric component (24200, 18015, 4015, 5015, 6015) is over molded onto said cannula post (24020, 18012, 4012, 5012) and said second elastomeric component (24220, 18115, 4115, 5115, 6115) is over molded into each of said clamping arms (24060, 24070, 18110, 18111, 4110, 4111, 5110, 5111, 6110, 6111).

5. The cannula (24000, 18000, 4000, 5000, 6000, 7000) of Claim 3, wherein said first elastomeric component (24200, 18015, 4015, 5015, 6015) is inserted onto said cannula post (24020, 18012, 4012, 5012) and said second elastomeric component (24220, 18115, 4115, 5115, 6115) is inserted into each of said clamping arms (24060, 24070, 18110, 18111, 4110, 4111, 5110, 5111, 6110, 6111).

6. The cannula (24000, 18000, 4000, 5000, 6000, 7000) of any of Claims 3 to 5, said clamping arms (24060, 24070, 18110, 18111, 4110, 4111, 5110, 5111, 6110, 6111) compressing said second elastomeric component (24220, 18115, 4115, 5115, 6115) against an outer surface of said vessel (24300, 18613) in turn compressing an inner surface of said vessel (24300, 18613) against said first elastomeric component (24200, 18015, 4015, 5015, 6015).

7. The cannula (24000, 18000, 4000, 5000, 6000, 7000) of any of Claims 3 to 6, wherein said cannula post (24020, 18012, 4012, 5012) has a flared base (24110) and said first elastomeric component (24200, 18015, 4015, 5015, 6015) has a flared end (24210) corresponding to the flared base (24110) of said cannula post (24020, 18012, 4012, 5012), and said clamping arms (24060, 24070, 18110, 18111, 4110, 4111, 5110, 5111, 6110, 6111) have a flared base (24120) and said second elastomeric component (24220, 18115, 4115, 5115, 6115) has a flared end (24230) corresponding to the flared base (24120) of said clamping arms (24060, 24070, 18110, 18111, 4110, 4111, 5110, 5111, 6110, 6111).

8. The cannula (24000, 18000, 4000, 5000, 6000, 7000) of Claim 7, said clamping arms (24060, 24070, 18110, 18111, 4110, 4111, 5110, 5111, 6110, 6111) compressing said flared end (24130) of said second elastomeric component (24220, 18115, 4115, 5115, 6115) against an outer surface of said vessel (24300, 18613) in turn compressing an inner surface of said vessel (24300, 18613) against said flared end (24210) of said first elastomeric component (24200, 18015, 4015, 5015, 6015).

9. The cannula (24000, 18000, 4000, 5000, 6000, 7000) of Claim 2, further comprising:
clamping detents (24150, 18125) on a first of said clamping arms (24060, 18110); and
clamping members (24130, 18120) on a second of said clamping arms (24070, 18111), wherein said clamping members (24130, 18120) engage with said clamping detents (24150, 18125) to retain said clamping arms (24060, 24070, 18110, 18111) in said clamped position.

10. The cannula (24000, 18000, 4000, 5000, 6000, 7000) of Claim 9, wherein pressure on said clamping arms (24060, 24070, 18110, 18111) causes them to disengagefrom said clamping detents (24150, 18125).

11. The cannula (24000, 18000, 4000, 5000, 6000, 7000) of any preceding claim, further comprising:
opposing shoulders (24050, 18040) extending from an outer surface of said cannula body (24010, 18010, 4010, 5010, 6010), said shoulders (24050, 18040) including a pivot slot (24260, 18041), a forward locking detent (24280) and a rearward locking detent (24270).

12. The cannula (24000, 18000, 4000, 5000, 6000, 7000) of Claim 11, further comprising:
a pivot bar (24090, 18046) and a locking bar (24100) at a rear end of each of said clamping arms (24060, 24070, 18110, 18111, 4110, 4111, 5110, 5111, 6110, 6111), said pivot bars (24090, 18046) pivotally received in said pivot slots (24260, 18041), said locking bars (24100) received and retained in said rearward locking detents (24270) when said clamping arms (24060, 24070, 18110, 18111, 4110, 4111, 5110, 5111, 6110, 6111) are in an open position, and said locking bars (24100) received and retained in said forward locking detents (24280) when said clamping arms (24060, 24070, 18110, 18111, 4110, 4111, 5110, 5111, 6110, 6111) are in a clamped position.

13. The cannula (24000, 18000, 4000, 5000, 6000, 7000) of Claim 12 when dependent on Claim 9, wherein said clamping members (24130, 18120), in said clamped position are slid rearwardly causing said locking bars (24100) to engage with said forward locking detents (24280).

14. The cannula (24000, 18000, 4000, 5000, 6000, 7000) of any preceding claim, wherein said tubing interface (24040, 18260, 4270, 5270, 6270, 7270) is an interface selected from the group consisting of: luer connector, barbed connector, locking connector, and spin lock type connector.

## Patentansprüche

1. Kanüle (24000, 18000, 4000, 5000, 6000, 7000) zum Verbinden eines Gefäßes (24300, 18613) eines zu perfundierenden Gewebes mit einem Perfusionskreislauf, umfassend:
einen Kanülenkörper (24010, 18010, 4010, 5010, 6010) mit einem Lumen (24030), das sich dort hindurch erstreckt;
eine Schlauchschnittstelle (24040, 18260, 4270, 5270, 6270, 7270) an einem ersten Ende des Kanülenkörpers (24010, 18010, 4010, 5010, 6010) für eine Verbindung mit dem Perfusionskreislauf; ein Kanülenstiel (24020, 18012, 4012, 5012) an einem zweiten Ende des Kanülenkörpers (24010, 18010, 4010, 5010, 6010), der konfiguriert ist, um das Gefäß (24300, 18613) des zu perfundierenden Gewebes aufzunehmen; und
zwei Klemmarme (24060, 24070, 18110, 18111, 4110, 4111, 5110, 5111, 6110, 6111), die mit dem Kanülenkörper (24010, 18010, 4010, 5010, 6010) schwenkbar verbunden und betätigbar sind, um mindestens ein Gefäß (24300, 18613) des zu perfundierenden Gewebes unter Kompression um den Kanülenstiel (24020, 18012, 4012, 5012) herum zu halten.

2. Kanüle (24000, 18000, 4000, 5000, 6000, 7000) nach Anspruch 1, wobei die zwei Klemmarme (24060, 24070, 18110, 18111, 4110, 4111, 5110, 5111, 6110, 6111) eine offene Position, wobei die Klemmarme (24060, 24070, 18110, 18111, 4110, 4111, 5110, 5111, 6110, 6111) relativ zu dem Kanülenkörper (24010, 18010, 4010, 5010, 6010) nach hinten geschwenkt sind, und eine geklemmte Position aufweisen, wobei die Klemmarme (24060, 24070, 18110, 18111, 4110, 4111, 5110, 5111, 6110, 6111) relativ zu dem Kanülenkörper (24010, 18010, 4010, 5010, 6010) nach vorne geschwenkt sind und miteinander um das Gefäß (24300, 18613) des zu perfundierenden Gewebes und den Kanülenstiel (24020, 18012, 4012, 5012) herum in Eingriff stehen.

3. Kanüle (24000, 18000, 4000, 5000, 6000, 7000) nach Anspruch 1 oder 2, ferner umfassend:
eine erste elastomere Komponente (24200, 18015, 4015, 5015, 6015), die um den Kanülenstiel (24020, 18012, 4012, 5012) herum positioniert ist; und
eine zweite elastomere Komponente (24220, 18115, 4115, 5115, 6115), die innerhalb jedes der Klemmarme (24060, 24070, 18110, 18111, 4110, 4111, 5110, 5111, 6110, 6111) positioniert ist.

4. Kanüle (24000, 18000, 4000, 5000, 6000, 7000) nach Anspruch 3, wobei die erste elastomere Komponente (24200, 18015, 4015, 5015, 6015) auf den Kanülenstiel (24020, 18012, 4012, 5012) überformt ist und die zweite elastomere Komponente (24220, 18115, 4115, 5115, 6115) in jeden der Klemmarme (24060, 24070, 18110, 18111, 4110, 4111, 5110, 5111, 6110, 6111) überformt ist.

5. Kanüle (24000, 18000, 4000, 5000, 6000, 7000) nach Anspruch 3, wobei die erste elastomere Komponente (24200, 18015, 4015, 5015, 6015) auf den Kanülenstiel (24020, 18012, 4012, 5012) aufgesetzt ist und die zweite elastomere Komponente (24220, 18115, 4115, 5115, 6115) in jeden der Klemmarme (24060, 24070, 18110, 18111, 4110, 4111, 5110, 5111, 6110, 6111) eingesetzt ist.

6. Kanüle (24000, 18000, 4000, 5000, 6000, 7000) nach einem der Ansprüche 3 bis 5, wobei die Klemmarme (24060, 24070, 18110, 18111, 4110, 4111, 5110, 5111, 6110, 6111) die zweite elastomere Komponente (24220, 18115, 4115, 5115, 6115) gegen eine Außenoberfläche des Gefäßes (24300, 18613) pressen, was wiederum eine Innenoberfläche des Gefäßes (24300, 18613) gegen die erste elastomere Komponente (24200, 18015, 4015, 5015, 6015) presst.

7. Kanüle (24000, 18000, 4000, 5000, 6000, 7000) nach einem der Ansprüche 3 bis 6, wobei der Kanülenstiel (24020, 18012, 4012, 5012) eine aufgeweitete Basis (24110) aufweist und die erste elastomere Komponente (24200, 18015, 4015, 5015, 6015) ein aufgeweitetes Ende (24210) aufweist, das der aufgeweiteten Basis (24110) des Kanülenstiels (24020, 18012, 4012, 5012) entspricht, und die Klemmarme (24060, 24070, 18110, 18111, 4110, 4111, 5110, 5111, 6110, 6111) eine aufgeweitete Basis (24120) aufweisen und die zweite elastomere Komponente (24220, 18115, 4115, 5115, 6115) ein aufgeweitetes Ende (24230) aufweist, das der aufgeweiteten Basis (24120) der Klemmarme (24060, 24070, 18110, 18111, 4110, 4111, 5110, 5111, 6110, 6111) entspricht.

8. Kanüle (24000, 18000, 4000, 5000, 6000, 7000) nach Anspruch 7, wobei die Klemmarme (24060, 24070, 18110, 18111, 4110, 4111, 5110, 5111, 6110, 6111) das aufgeweitete Ende (24130) der zweiten elastomeren Komponente (24220, 18115, 4115, 5115, 6115) gegen eine Außenoberfläche des Gefäßes (24300, 18613) pressen, was wiederum eine Innenoberfläche des Gefäßes (24300, 18613) gegen das aufgeweitete Ende (24210) der ersten elastomeren Komponente (24200, 18015, 4015, 5015, 6015) presst.

9. Kanüle (24000, 18000, 4000, 5000, 6000, 7000) nach Anspruch 2, ferner umfassend:
Klemmrastungen (24150, 18125) an einem ersten der Klemmarme (24060, 18110); und
Klemmelemente (24130, 18120) an einem zweiten der Klemmarme (24070, 18111), wobei die Klemmelemente (24130, 18120) mit den Klemmrastungen (24150, 18125) in Eingriff stehen, um die Klemmarme (24060, 24070, 18110, 18111) in der geklemmten Position zu halten.

10. Kanüle (24000, 18000, 4000, 5000, 6000, 7000) nach Anspruch 9, wobei Druck auf die Klemmarme (24060, 24070, 18110, 18111) bewirkt, dass sie sich von den Klemmrastungen (24150, 18125) lösen.

11. Kanüle (24000, 18000, 4000, 5000, 6000, 7000) nach einem der vorstehenden Ansprüche, ferner umfassend:
gegenüberliegende Schultern (24050, 18040), die sich von einer Außenoberfläche des Kanülenkörpers (24010, 18010, 4010, 5010, 6010) erstrecken, wobei die Schultern (24050, 18040) einen Schwenkschlitz (24260, 18041), eine vordere Verriegelungsrastung (24280) und eine hintere Verriegelungsrastung (24270) einschließen.

12. Kanüle (24000, 18000, 4000, 5000, 6000, 7000) nach Anspruch 11, ferner umfassend:
eine Schwenkstange (24090, 18046) und eine Verriegelungsstange (24100) an einem hinteren Ende jedes der Klemmarme (24060, 24070, 18110, 18111, 4110, 4111, 5110, 5111, 6110, 6111), wobei die Schwenkstangen (24090, 18046) in den Schwenkschlitzen (24260, 18041) schwenkbar aufgenommen sind, wobei die Verriegelungsstangen (24100) in den hinteren Verriegelungsrastungen (24270) aufgenommen und gehalten werden, wenn sich die Klemmarme (24060, 24070, 18110, 18111, 4110, 4111, 5110, 5111, 6110, 6111) in einer offenen Position befinden, und die Verriegelungsstangen (24100) in den vorderen Verriegelungsrastungen (24280) aufgenommen und gehalten werden, wenn sich die Klemmarme (24060, 24070, 18110, 18111, 4110, 4111, 5110, 5111, 6110, 6111) in einer geklemmten Position befinden.

13. Kanüle (24000, 18000, 4000, 5000, 6000, 7000) nach Anspruch 12, wenn abhängig von Anspruch 9, wobei die Klemmelemente (24130, 18120) in der geklemmten Position nach hinten verschoben werden, was bewirkt, dass die Verriegelungsstangen (24100) in die vorderen Verriegelungsrastungen (24280) eingreifen.

14. Kanüle (24000, 18000, 4000, 5000, 6000, 7000) nach einem der vorstehenden Ansprüche, wobei die Schlauchschnittstelle (24040, 18260, 4270, 5270, 6270, 7270) eine Schnittstelle ist, die aus der Gruppe ausgewählt ist, bestehend aus: Luer-Verbinder, Widerhakenverbinder, Verriegelungsverbinder und Verbinder vom Spin-Lock-Typ.

## Revendications

1. Canule (24000, 18000, 4000, 5000, 6000, 7000) destinée à relier un vaisseau (24300, 18613) d'un tissu à perfuser à une boucle de perfusion, comprenant :
un corps (24010, 18010, 4010, 5010, 6010) de canule comportant une lumière (24030) s'étendant à travers celui-ci ;
une interface de tubulure (24040, 18260, 4270, 5270, 6270, 7270) au niveau d'une première extrémité du corps (24010, 18010, 4010, 5010, 6010) de canule pour la liaison avec la boucle de perfusion ;un montant (24020, 18012, 4012, 5012) de canule au niveau d'une seconde extrémité du corps (24010, 18010, 4010, 5010, 6010) de canule, conçu pour recevoir le vaisseau (24300, 18613) du tissu à perfuser ; et
deux bras de serrage (24060, 24070, 18110, 18111, 4110, 4111, 5110, 5111, 6110, 6111) reliés de manière pivotante audit corps (24010, 18010, 4010, 5010, 6010) de canule et pouvant fonctionner afin de retenir au moins un vaisseau (24300, 18613) du tissu à perfuser en compression autour du montant (24020, 18012, 4012, 5012) de canule.

2. Canule (24000, 18000, 4000, 5000, 6000, 7000) selon la revendication 1, dans laquelle lesdits deux bras de serrage (24060, 24070, 18110, 18111, 4110, 4111, 5110, 5111, 6110, 6111) présentent une position ouverte, dans laquelle les bras de serrage (24060, 24070, 18110, 18111, 4110, 4111, 5110, 5111, 6110, 6111) sont pivotés vers l'arrière par rapport audit corps (24010, 18010, 4010, 5010, 6010) de canule, et une position serrée, dans laquelle les bras de serrage (24060, 24070, 18110, 18111, 4110, 4111, 5110, 5111, 6110, 6111) ont pivoté vers l'avant par rapport audit corps (24010, 18010, 4010, 5010, 6010) de canule et sont en prise l'un avec l'autre autour dudit vaisseau (24300, 18613) du tissu à perfuser et du montant (24020, 18012, 4012, 5012) de canule.

3. Canule (24000, 18000, 4000, 5000, 6000, 7000) selon la revendication 1 ou la revendication 2, comprenant en outre :
un premier composant élastomère (24200, 18015, 4015, 5015, 6015) positionné autour dudit montant (24020, 18012, 4012, 5012) de canule ; et
un second composant élastomère (24220, 181 15, 41 15, 5115, 61 15) positionné à l'intérieur de chacun desdits bras de serrage (24060, 24070, 18110, 18111, 4110, 4111, 5110, 5111, 6110, 6111).

4. Canule (24000, 18000, 4000, 5000, 6000, 7000) selon la revendication 3, dans laquelle ledit premier composant élastomère (24200, 18015, 4015, 5015, 6015) est surmoulé sur ledit montant (24020, 18012, 4012, 5012) de canule et ledit second composant élastomère (24220, 18115, 4115, 5115, 6115) est surmoulé dans chacun desdits bras de serrage (24060, 24070, 18110, 18111, 4110, 4111, 5110, 5111, 6110, 6111).

5. Canule (24000, 18000, 4000, 5000, 6000, 7000) selon la revendication 3, dans laquelle ledit premier composant élastomère (24200, 18015, 4015, 5015, 6015) est inséré sur ledit montant (24020, 18012, 4012, 5012) de canule et ledit second composant élastomère (24220, 18115, 4115, 5115, 6115) est inséré dans chacun desdits bras de serrage (24060, 24070, 18110, 18111, 4110, 4111, 5110, 5111, 6110, 6111).

6. Canule (24000, 18000, 4000, 5000, 6000, 7000) selon l'une quelconque des revendications 3 à 5, lesdits bras de serrage (24060, 24070, 18110, 18111, 4110, 4111, 5110, 5111, 6110, 6111) comprimant ledit second composant élastomère (24220, 18115, 4115, 5115, 6115) contre une surface externe dudit vaisseau (24300, 18613), comprimant à son tour une surface interne dudit vaisseau (24300, 18613) contre ledit premier composant élastomère (24200, 18015, 4015, 5015, 6015).

7. Canule (24000, 18000, 4000, 5000, 6000, 7000) selon l'une quelconque des revendications 3 à 6, dans laquelle ledit montant (24020, 18012, 4012, 5012) de canule présente une base évasée (24110) et ledit premier composant élastomère (24200, 18015, 4015, 5015, 6015) présente une extrémité évasée (24210) correspondant à la base évasée (24110) dudit montant (24020, 18012, 4012, 5012) de canule et lesdits bras de serrage (24060, 24070, 18110, 18111, 4110, 4111, 5110, 5111, 6110, 6111) présentent une base évasée (24120) et ledit second composant élastomère (24220, 18115, 4115, 5115, 6115) présente une extrémité évasée (24230) correspondant à la base évasée (24120) desdits bras de serrage (24060, 24070, 18110, 18111, 4110, 4111, 5110, 5111, 6110, 6111).

8. Canule (24000, 18000, 4000, 5000, 6000, 7000) selon la revendication 7, lesdits bras de serrage (24060, 24070, 18110, 18111, 4110, 4111, 5110, 5111, 6110, 6111) comprimant ladite extrémité évasée (24130) dudit second composant élastomère (24220, 18115, 4115, 5115, 6115) contre une surface externe dudit vaisseau (24300, 18613), comprimant à son tour une surface interne dudit vaisseau (24300, 18613) contre ladite extrémité évasée (24210) dudit premier composant élastomère (24200, 18015, 4015, 5015, 6015).

9. Canule (24000, 18000, 4000, 5000, 6000, 7000) selon la revendication 2, comprenant en outre :
des crans de serrage (24150, 18125) sur un premier desdits bras de serrage (24060, 18110) ; et
des éléments de serrage (24130, 18120) sur un second desdits bras de serrage (24070, 18111), dans laquelle lesdits éléments de serrage (24130, 18120) viennent en prise avec lesdits crans de serrage (24150, 18125) afin de retenir lesdits bras de serrage (24060, 24070, 18110, 18111) dans ladite position serrée.

10. Canule (24000, 18000, 4000, 5000, 6000, 7000) selon la revendication 9, dans laquelle une pression sur lesdits bras de serrage (24060, 24070, 18110, 18111) les amène à se désolidariser desdits crans de serrage (24150, 18125).

11. Canule (24000, 18000, 4000, 5000, 6000, 7000) selon l'une quelconque revendication précédente, comprenant en outre :
des épaulements (24050, 18040) opposés s'étendant à partir d'une surface externe dudit corps (24010, 18010, 4010, 5010, 6010) de canule, lesdits épaulements (24050, 18040) comportant une fente à pivot (24260, 18041), un cran de verrouillage avant (24280) et un cran de verrouillage arrière (24270).

12. Canule (24000, 18000, 4000, 5000, 6000, 7000) selon la revendication 11, comprenant en outre :
une barre de pivot (24090, 18046) et une barre de verrouillage (24100) au niveau d'une extrémité arrière de chacun desdits bras de serrage (24060, 24070, 18110, 18111, 4110, 4111, 5110, 5111, 6110, 6111), lesdites barres de pivot (24090, 18046) étant logées de manière pivotante dans lesdites fentes à pivot (24260, 18041), lesdites barres de verrouillage (24100) étant logées et retenues dans lesdits crans de verrouillage arrière (24270) lorsque lesdits bras de serrage (24060, 24070, 18110, 18111, 4110, 4111, 5110, 5111, 6110, 6111) sont dans une position ouverte et lesdites barres de verrouillage (24100) étant logées et retenues dans lesdits crans de verrouillage avant (24280) lorsque lesdits bras de serrage (24060, 24070, 18110, 18111, 4110, 4111, 5110, 5111, 6110, 6111) sont dans une position serrée.

13. Canule (24000, 18000, 4000, 5000, 6000, 7000) selon la revendication 12 lorsqu'elle dépend de la revendication 9, dans laquelle lesdits éléments de serrage (24130, 18120), dans ladite position serrée, sont coulissés vers l'arrière, amenant lesdites barres de verrouillage (24100) à venir en prise avec lesdits crans de verrouillage avant (24280).

14. Canule (24000, 18000, 4000, 5000, 6000, 7000) selon une quelconque revendication précédente, dans laquelle ladite interface de tubulure (24040, 18260, 4270, 5270, 6270, 7270) est une interface choisie dans le groupe constitué de : raccord Luer, raccord cannelé, raccord à verrouillage et raccord de type spinlock.
